# EUROPEAN PATENT APPLICATION

(11) **EP 3 540 686 A2**
(43) Date of publication of application: **18.09.2019**
(21) Application number: 19160498.2
(22) Date of filing: 04.03.2019
(51) Int. Cl.: G06T 5/00, G06T 7/55

(54) **MEASURING APPARATUS, MEASURING SYSTEM, MEASURING METHOD, AND CARRIER MEANS STORING PROGRAM CODE**

(30) Priority: 14.03.2018 JP 2018047336; 11.05.2018 JP 2018092316
(71) Applicant: Ricoh Company, Ltd., Tokyo 143-8555 (JP)
(72) Inventor: NAKAMURA, Satoshi, Ohta-ku, Tokyo 143-8555 (JP); NIYAGAWA, Kazuya, Ohta-ku, Tokyo 143-8555 (JP)
(74) Representative: White, Duncan Rohan

(57) **Abstract**

A measuring apparatus (10), a measuring system (1), a measuring method, and a carrier means carrying computer readable code. The measuring apparatus (10) and the measuring method include obtaining data indicating position coordinates of a surface of an object (30), and outputting information to associate amount information of the data belonging to at least one of a plurality of subspaces (401, 402, 403, 404, 411, 412, 413, 414, 415, 416, 417, 418) obtained by dividing a coordinate space with the plurality of subspaces (401, 402, 403, 404, 411, 412, 413, 414, 415, 416, 417, 418) when the amount information and the plurality of subspaces (401, 402, 403, 404, 411, 412, 413, 414, 415, 416, 417, 418) are displayed. The measuring system (1) includes a measuring device (20) and the measuring apparatus (10). The code that is carried by the carrier means controls a computer system to carry out the measuring method.

## Description

### BACKGROUND

### Technical Field

Embodiments of the present disclosure relate to a measuring apparatus, a measuring system, a measuring method, and carrier means storing program code.

### Background Art

When the shape of a three-dimensional object (such a three-dimensional object may be referred to simply as an object in the following description) is measured using a three-dimensional measuring device, as known in the art, the entirety of the object cannot be measured all at once. This is because there is a subarea that cannot be observed from a single viewpoint due to occlusion. A plurality of three-dimensional measuring devices may be arranged so as to surround the object, and the object may be omnidirectionally measured all at once. The size of an apparatus becomes so large in most cases that the application is limited due to restrictions on, for example, installation site and price. For this reason, in most cases, while changing the relative positions of an object and a three-dimensional measuring device, the object is measured from various kinds of points and directions upon dividing the object into several subareas. Then, the measurement results are combined to obtain the measurement of the entirety of the object. In so doing, the person who performs the measurement needs to proceed with operation upon determining the positions to be measured next and directions, while sequentially checking, for example, whether the number of groups of points obtained by performing shape measurement (i.e., the amount of shape measurement data), the distribution of the points, or the density of the points is sufficient.

Technologies are known in the art in which how well the three-dimensional shape data indicating the measured portions of the object can be viewed when the three-dimensional shape data is viewed from a plurality of viewpoints is calculated and a viewpoint is selected that best satisfies predetermined criteria, after which the three-dimensional shape data obtained from the selected viewpoint is presented to the person who performs the measurement (technician)the person who performs the measurement (see JP-2014-010559-A).

In such shape measurement of a three-dimensional object as above using a three-dimensional measuring device, the determination as to whether the number of groups of points obtained by performing shape measurement (i.e., the amount of shape measurement data) is sufficient tends to be affected by a subjective point of view of a person who performs measurement (technician), and its criteria for judgment varies depending on the intended use. When a person who performs measurement is different from a user of the measurement results and a lack of shape measurement data or variations in the number of points are found at the stage where the user uses the measurement results, the process needs to be returned to the previous step due to the remeasurement. In particular, when a pen-shaped three-dimensional measuring device (such a three-dimensional measuring device may be referred to as a digitizer in the following description) is used to measure the shape of an object, the number of groups of points that is obtained in one-time measurement is small and its distribution is also very sparse compared with a three-dimensional measuring device that can measure a wide area at once (such a three-dimensional measuring device may be referred to as a three-dimensional scanner in the following description) such as a laser scanner. Due to this configuration, in order to improve the efficiency of the shape measurement of a three-dimensional object, a person who performs measurement needs to be assisted such that he or she can measure the minimum necessary points as completely as possible. In the disclosure of JP-2014-010559-A, the criteria for choosing a viewpoint are based on the proportion of maximum values for the degree of how well an object can be viewed, and are not necessarily based on whether the shape measurement data is sufficient. Further, if a person who performs measurement (technician) is only provided with a particular viewpoint, he or she cannot intuitively figure out how far the shape measurement is progressing in the entirety of an object to be measured.

### SUMMARY

Embodiments of the present disclosure described herein provide a measuring apparatus, a measuring system, a measuring method, and carrier means carrying computer readable code. The measuring apparatus and the measuring method involve obtaining data indicating position coordinates of a surface of an object and outputting information to associate amount information of the data belonging to at least one of a plurality of subspaces obtained by dividing a coordinate space with the plurality of subspaces when the amount information and the plurality of subspaces are displayed. The measuring system includes a measuring device and the measuring apparatus. The the code that is carried by the carrier means controls a computer system to carry out the measuring method.

According to one aspect of the present disclosure, a person who performs measurement can easily figure out whether the shape measurement data obtained by measuring the shape of an object is sufficient.

### BRIEF DESCRIPTION OF THE DRAWINGS

A more complete appreciation of embodiments and the many attendant advantages thereof will be readily obtained as the same becomes better understood by reference to the following detailed description when considered in connection with the accompanying drawings.
FIG. 1 is a diagram illustrating an overall configuration of a measurement support system according to a first embodiment of the present disclosure.
FIG. 2 is a diagram illustrating a hardware configuration of a measurement support device according to the first embodiment of the present disclosure.
FIG. 3 is a diagram illustrating a configuration of the functional blocks of a measurement support device according to the first embodiment of the present disclosure.
FIG. 4 is a diagram illustrating an example case in which coordinate space is divided into a plurality of subspaces, according to the first embodiment of the present disclosure.
FIG. 5 is a diagram illustrating an example case in which a three-dimensionally measured object is color-coded on a subarea-by-subarea basis, according to the first embodiment of the present disclosure.
FIG. 6 is a diagram illustrating an example graph in which the evaluation value of each subarea of a three-dimensionally measured object is indicated, according to the first embodiment of the present disclosure.
FIG. 7 is a flowchart of the processes performed by a measurement support device according to the first embodiment of the present disclosure.
FIG. 8 is a diagram illustrating an example case in which a coordinate system is set based on the distribution of shape measurement data, according to a modification of the first embodiment of the present disclosure.
FIG. 9 is a diagram illustrating a method of setting a reference point of a head according to the second embodiment of the present disclosure.
FIG. 10 is a diagram illustrating an example case in which coordinate space is divided into a plurality of subspaces, according to the second embodiment of the present disclosure.
FIG. 11 is a diagram illustrating an example case in which a three-dimensionally measured object is color-coded on a subarea-by-subarea basis, according to the second embodiment of the present disclosure.
FIG. 12 is a diagram illustrating an example case in which the two-dimensionally projected image of a measured object of an object is color-coded, in a modification of the second embodiment of the present disclosure.
FIG. 13 is a diagram illustrating a configuration of the functional blocks of a measurement support device according to a third embodiment of the present disclosure.
FIG. 14 is a diagram illustrating how vectors are set and how noise determination processes are performed in the third embodiment of the present disclosure.
FIG. 15 is a flowchart of the processes performed by a measurement support device according to the third embodiment of the present disclosure.
FIG. 16 is a diagram illustrating how vectors are set and how noise determination processes are performed in a first modification of the third embodiment of the present disclosure.
FIG. 17 is a diagram illustrating a configuration of the functional blocks of a measurement support device according to a second modification of the third embodiment of the present disclosure.
FIG. 18 is a diagram illustrating how vectors are set and how noise determination processes are performed in the second modification of the third embodiment of the present disclosure.
FIG. 19 is a flowchart of the processes performed by a measurement support device according to the second modification of the third embodiment of the present disclosure.

The accompanying drawings are intended to depict embodiments of the present disclosure and should not be interpreted to limit the scope thereof. The accompanying drawings are not to be considered as drawn to scale unless explicitly noted.

### DETAILED DESCRIPTION

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "includes" and/or "including", when used in this specification, specify the presence of stated features, integers, steps, operations, elements, and/or components, but do not preclude the presence or addition of one or more other features, integers, steps, operations, elements, components, and/or groups thereof.

In describing example embodiments shown in the drawings, specific terminology is employed for the sake of clarity. However, the present disclosure is not intended to be limited to the specific terminology so selected and it is to be understood that each specific element includes all technical equivalents that have the same structure, operate in a similar manner, and achieve a similar result.

In the following description, illustrative embodiments will be described with reference to acts and symbolic representations of operations (e.g., in the form of flowcharts) that may be implemented as program modules or functional processes including routines, programs, objects, components, data structures, etc., that perform particular tasks or implement particular abstract data types and may be implemented using existing hardware at existing network elements or control nodes. Such existing hardware may include one or more central processing units (CPUs), digital signal processors (DSPs), application-specific-integrated-circuits (ASICs), field programmable gate arrays (FPGAs), computers or the like. These terms in general may be collectively referred to as processors.

Unless specifically stated otherwise, or as is apparent from the discussion, terms such as "processing" or "computing" or "calculating" or "determining" or "displaying" or the like, refer to the action and processes of a computer system, or similar electronic computing device, that manipulates and transforms data represented as physical, electronic quantities within the computer system's registers and memories into other data similarly represented as physical quantities within the computer system memories or registers or other such information storage, transmission or display devices.

A measuring apparatus, a measuring system, a measuring method, and a recording medium storing program code according to an embodiment of the present disclosure are described below in detail with reference to the drawings. Numerous additional modifications and variations are possible in light of the above teachings. It is therefore to be understood that within the scope of the appended claims, the disclosure of the present disclosure may be practiced otherwise than as specifically described herein. For example, elements and/or features of different illustrative embodiments may be combined with each other and/or substituted for each other within the scope of this disclosure and appended claims.

### First Embodiment

### Overall Configuration of Measurement Support System

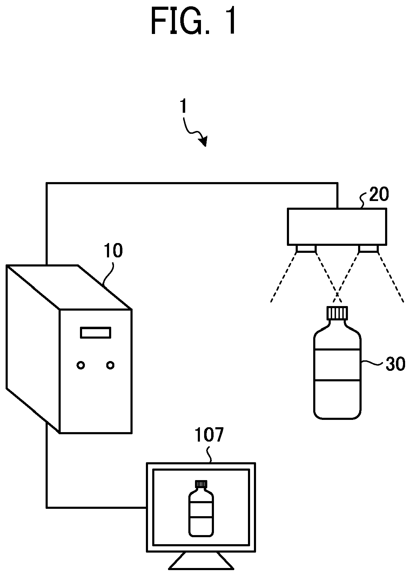

FIG. 1 is a diagram illustrating an overall configuration of a measurement support system 1 according to a first embodiment of the present disclosure.

As illustrated in FIG. 1, the measurement support system 1 according to the present embodiment includes a measurement support device 10 (including a display 107) and a three-dimensional measuring device 20 (an example of measuring device).

The three-dimensional measuring device 20 measures the surface shape of an object 30, which is a three-dimensionally measured object, to obtain the shape measurement data that makes up the surface shape of the object 30. Note that the shape measurement data consists of dots. A person who performs measurement measures the surface shape of the object 30 while changing the position and direction of the three-dimensional measuring device 20. For example, the shape measurement data is the data of the three-dimensional position of a specific point on the surface of the object 30. In order for the three-dimensional position of the specific point to be specified, a predetermined three-dimensional coordinate system need to be set. As long as it is a device that can measure shape measurement data, the three-dimensional measuring device 20 may be any desired device. For example, the three-dimensional measuring device 20 may be a laser scanner (three-dimensional scanner) that irradiates an object with a laser beam to measure the distance based on the length of time it takes for the reflected light to return from the object, a stereo camera that estimates the distance based on disparities between a pair of images captured by two separate cameras, a range sensor using active stereo methods that projects pattern light with known structure onto a target object to estimate the distance based on the deflection of the pattern light, a digitizer that measures the distance by detecting the electromagnetic field caused by a source using a stylus whose tip is filled with a coil, and a multi-view stereo device that combines the images captured from multiple views by a monocular camera to measure the surface shape.

The measurement support device 10 is an information processing apparatus that obtains the shape measurement data of the surface shape of the object 30 measured by the three-dimensional measuring device 20 and supports the measurement by presenting the obtained data to a person who performs measurement (displaying the obtained data on the display 107) such that the amount of obtained shape measurement data (i.e., the number of groups of points) can visually be figured out by that user. For example, the measurement support device 10 is an information processing apparatus such as a personal computer (PC) or a workstation, or a tablet PC. When the shape measurement data can be measured using the camera function provided for a smartphone, such a smartphone may be used as the measurement support device 10 and the three-dimensional measuring device 20.

### Hardware Configuration of Measurement Support Device

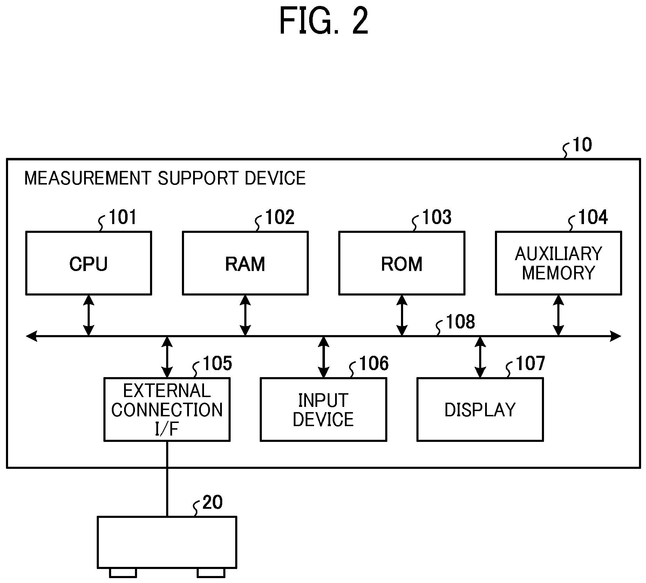

FIG. 2 is a diagram illustrating a hardware configuration of the measurement support device 10 according to the first embodiment of the present disclosure.

A hardware configuration of the measurement support device 10 according to the present embodiment is described below with reference to FIG. 2.

As illustrated in FIG. 2, the measurement support device 10 is provided with a central processing unit (CPU) 101, a random access memory (RAM) 102, a read only memory (ROM) 103, an auxiliary memory 104, an external connection interface (I/F) 105, an input device 106, and a display 107.

The CPU 101 causes the operation of the measurement support device 10 in a centralized manner. More specifically, the CPU 101 uses the RAM 102 as a work area, and executes a program stored in, for example, the ROM 103 or the auxiliary memory 104. By so doing, the entire operation of the measurement support device 10 is controlled.

For example, the RAM 102 is a volatile memory that is used as a work area of the CPU 101. For example, the ROM 103 is a nonvolatile memory that stores, for example, various kinds of settings and a program to be executed when the measurement support device 10 is started.

For example, the auxiliary memory 104 is a nonvolatile storage device that stores various kinds of data such as an operating system (OS), an application program, and the shape measurement data of a measured object. The auxiliary memory 104 is a memory such as a hard disk drive (HDD), a solid state disk (SSD), a flash memory, or an optical disk, in which data can electrically, magnetically, or optically be stored.

The external connection interface 105 is a communication interface that is coupled to an external device such as the three-dimensional measuring device 20. For example, the external connection interface 105 is a universal serial bus (USB) interface.

The input device 106 is an input device through which, for example, texts or numbers are input, various kinds of instructions are selected, and a cursor or pointer is moved. For example, the input device 106 may be a mouse or a keyboard.

The display 107 is a display interface that displays, for example, texts, numbers, various kinds of screen images, an image of a three-dimensionally measured object. For example, the display 107 may be a cathode-ray tube (CRT) display, a liquid crystal display (LCD), or an organic electroluminescence (EL) display.

When the measurement support device 10 is an information processing device such as a tablet personal computer (PC), for example, the input device 106 and the display 107 may be configured by a liquid crystal display (LCD) provided with touch panel functionality. In such cases, the input device 106 is implemented by touch input function of the touch panel functionality, and the display 107 is implemented by display function of the touch panel functionality.

The bus 108 is a transmission line that connects the above elements to each other and transmits, for example, address signals, data signals, and various kinds of control signals.

Note that the hardware configuration of the measurement support device 10 as illustrated in FIG. 2 is given by way of example, and it is not necessary for the measurement support device 10 to include all the elements illustrated in FIG. 2. Alternatively, the measurement support device 10 may further include other elements not specifically described herein.

### Operations and Configuration of Functional Blocks of Measurement Support Device

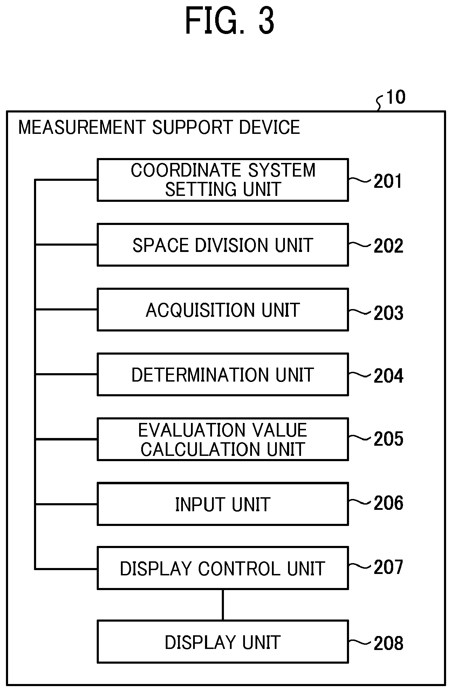

FIG. 3 is a diagram illustrating a configuration of the functional blocks of the measurement support device 10 according to the first embodiment of the present disclosure.

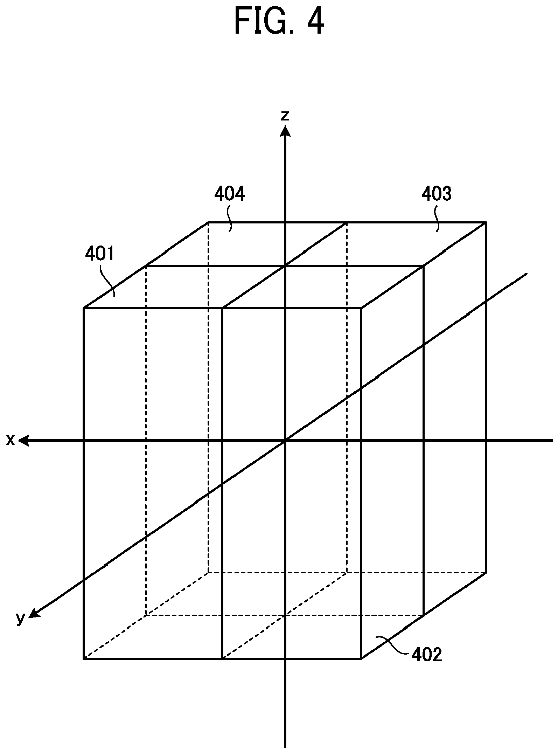

FIG. 4 is a diagram illustrating an example case in which coordinate space is divided into a plurality of subspaces, according to the first embodiment the present disclosure.

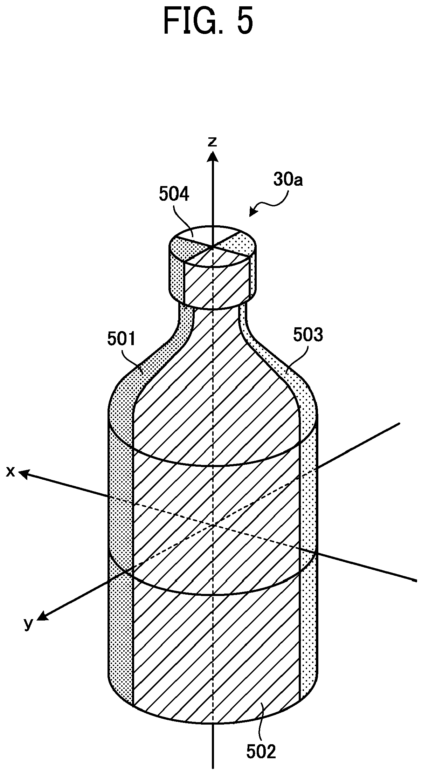

FIG. 5 is a diagram illustrating an example case in which a three-dimensionally measured object 30a, which corresponds to the object 30, is color-coded on a subarea-by-subarea basis, according to the first embodiment of the present disclosure.

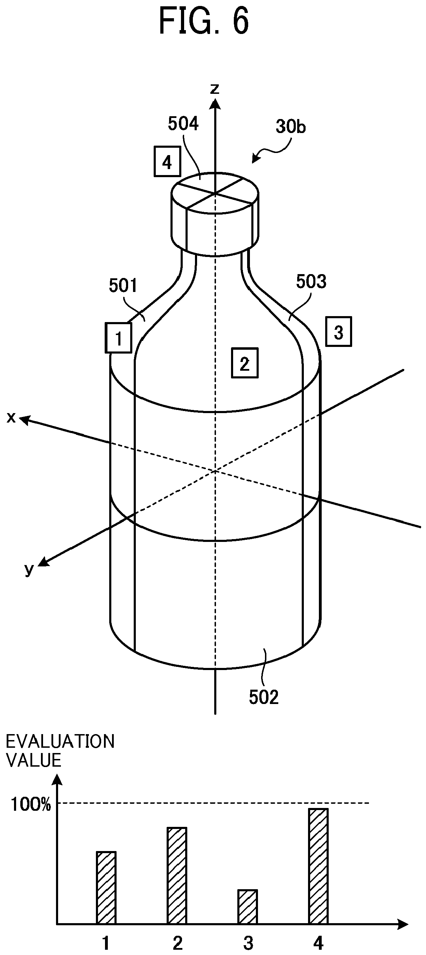

FIG. 6 is a diagram illustrating an example graph in which the evaluation value of each subarea of a three-dimensionally measured object 30b, which corresponds to the object 30, is indicated, according to the first embodiment of the present disclosure.

Operations and a configuration of functional blocks of the measurement support device 10 according to the present embodiment are described below with reference to FIG. 3 to FIG. 6.

As illustrated in FIG. 3, the measurement support device 10 includes a coordinate system setting unit 201 (construction unit), a space division unit 202 (division unit), an acquisition unit 203, a determination unit 204, an evaluation value calculation unit 205 (calculation unit), an input unit 206, a display control unit 207 (control unit), and a display unit 208.

For example, the coordinate system setting unit 201 is a functional unit that sets (constructs) three-dimensional coordinate space (an example of coordinate space) based on a predetermined reference point. For example, the coordinate system setting unit 201 may set coordinate space obtained by performing linear transformation inside the three-dimensional coordinate space that the three-dimensional measuring device 20 has internally. Alternatively, the coordinate system setting unit 201 may set coordinate space by moving or rotating the three-dimensional coordinate space. For example, the coordinate system setting unit 201 may be implemented by a program executed by the CPU 101 as illustrated in FIG. 2.

A person who performs measurement may control the measurement support device 10 to explicitly specify a reference point that the coordinate system setting unit 201 uses to set three-dimensional coordinate space. When a rough shape of the object 30 is known, preliminarily set points may be used. Preferably, a characteristic point in the shape of the object 30 is used as a reference point. For example, when the object 30 is in a conical shape, the vertex or a point of intersection where a diameter of the base intersects the perimeter of a circle may be used as a reference point. Alternatively, when the object 30 is in a car, for example, the center of the front bumper or the rear bumper or edges of the right and left mirrors may be used. If the object 30 is an asymmetrical object, the center of the object 30 or a point near the center of gravity may be used as a reference point.

The space division unit 202 is a functional unit that divides the three-dimensional coordinate space set by the coordinate system setting unit 201 into a plurality of subspaces. For example, the space division unit 202 may divide three-dimensional coordinate space according to the number of divisions, a to-be-separated position, and a way of division, which are set in advance. The space division unit 202 may conceptually divide the entirety of the three-dimensional coordinate space set by the coordinate system setting unit 201 into a plurality of subspaces. Alternatively, the three-dimensional coordinate space may be divided into a plurality of subspaces within the range of space where the three-dimensional coordinate space to which the three-dimensionally measured object of the object 30 is set is predicted to exist.

By way of example, FIG. 4 illustrates an example case in which three-dimensional coordinate space constructed with XYZ axes is divided into subspaces 401 to 404 by the space division unit 202. As illustrated in FIG. 4, the subspace 401 is space corresponding to the first quadrant of the xy plane, and the subspace 402 is space corresponding to the second quadrant. In a similar manner, the subspace 403 is space corresponding to the third quadrant, and the subspace 404 is space corresponding to the fourth quadrant.

For example, the space division unit 202 may be implemented by a program executed by the CPU 101 as illustrated in FIG. 2.

The acquisition unit 203 is a functional unit that obtains the shape measurement data of the object 30 measured by the three-dimensional measuring device 20. The acquisition unit 203 stores the obtained shape measurement data in the auxiliary memory 104. For example, the acquisition unit 203 may be implemented by the external connection interface 105 as illustrated in FIG. 2 and a program executed by the CPU 101 as illustrated in FIG. 2.

The determination unit 204 is a functional unit that determines to which one of the subspaces of the three-dimensional coordinate space each part (or each area) of the three-dimensionally measured object represented by the shape measurement data (dot data) of the object 30 obtained by the acquisition unit 203 belongs. In the present embodiment, a part of the three-dimensionally measured object that is determined by the determination unit 204 to belong to a particular subspace may be referred to as a subarea. In other words, the determination unit 204 determines to which one of the subspaces each part of the three-dimensionally measured object belongs. As a result, the three-dimensionally measured object is divided into a plurality of subareas.

By way of example, a three-dimensionally measured object 30a (an example of a measured object) that is represented by the shape measurement data of the object 30 obtained by the acquisition unit 203 is illustrated in FIG. 5. In the present embodiment, as illustrated in FIG. 4 as above, it is assumed that three-dimensional coordinate space is divided into the subspaces 401 to 404, and the three-dimensionally measured object 30a is included in the space configured by the subspaces 401 to 404. The determination unit 204 determines that a subarea 501 of the three-dimensionally measured object 30a belongs to the subspace 401, and determines that a subarea 502 of the three-dimensionally measured object 30a belongs to the subspace 402. The determination unit 204 determines that a subarea 503 of the three-dimensionally measured object 30a belongs to the subspace 403, and determines that a subarea 504 of the three-dimensionally measured object 30a belongs to the subspace 404.

For example, the determination unit 204 may be implemented by a program executed by the CPU 101 as illustrated in FIG. 2.

The evaluation value calculation unit 205 is a functional unit that calculates and obtains the evaluation value of a subspace based on the shape measurement data included in that subspace, i.e., the shape measurement data that makes up a subarea of the three-dimensionally measured object included in the subspace.

The evaluation values that are calculated by the evaluation value calculation unit 205 may include, for example, the integrated value of the amount of shape measurement data (i.e., the number of points) that belongs to a subspace. The ratio of the integrated values of the amount of all the shape measurement data (i.e., the number of points) obtained in that subspace may be calculated with reference to a reference value for a predetermined amount (i.e., a predetermined number of points) set for each one of the subspaces, and the obtained ratio may be used as an evaluation value. Alternatively, the density of the shape measurement data belonging to in the subspace may be used as an evaluation value. In such cases, the actual ratio of density of the obtained subspaces may be calculated with reference to a reference value for a predetermined density set for each one of the subspaces, and the obtained ratio may be used as an evaluation value. Alternatively, the subspaces may be divided into even smaller subspaces, and the density may be calculated for each one of the even smaller subspaces, and the evaluation value of the subspace may be calculated based on its maximum density or the minimum density. Such evaluation values as listed above are an example of information about the amount of the shape measurement data that makes up the subareas of a measured object.

For example, the evaluation value calculation unit 205 may be implemented by a program executed by the CPU 101 as illustrated in FIG. 2.

The input unit 206 is a functional unit that accepts operation or input performed by a person who performs measurement. For example, the input unit 206 accepts the operation or input (for example, a dragging operation or a swiping operation made by a mouse) of changing the viewpoint of the three-dimensionally measured object of the object 30, which is being displayed on the display unit 208. The input unit 206 may be implemented by the input device 106 as illustrated in FIG. 2.

The display control unit 207 is a functional unit that causes the display operation of the display unit 208. More specifically, the display control unit 207 causes the display unit 208 to display a three-dimensionally measured object represented by the shape measurement data obtained by the acquisition unit 203. In so doing, the display control unit 207 causes a display to display the three-dimensionally measured object in visual presentation where a person who performs measurement can easily and intuitively figure out the situation, based on the evaluation value of the subspace that corresponds to each subarea of the three-dimensionally measured object.

As illustrated in FIG. 5, for example, the display control unit 207 causes a display to display the subareas of the three-dimensionally measured object 30a (i.e., the subareas 501 to 504 in FIG. 5) belonging to the multiple subspaces upon color-coding these subareas with different colors, based on the level of evaluation value of each subspace. As a coloring method, for example, dot-shaped objects that indicate the positions of the shape measurement data (dot data) that makes up a three-dimensionally measured object may be colored, or planes that are formed by the line segments connecting the points of the obtained shape measurement data may be colored. As a color-coding method, for example, the green color is emphasized as the evaluation value is higher and the red color is emphasized as the evaluation value is lower, and the yellow color is emphasized when the evaluation value is in the middle. Moreover, as the shape measurement data is added by the person who performs the measurement, the evaluation values improve, and the color is changed from red to yellow, and then changed to green. As the subareas are color-coded based on the evaluation values as described above, can instantly figure out whether the shape measurement data of each part of the object 30 is sufficient.

For example, the display control unit 207 may be implemented by a program executed by the CPU 101 as illustrated in FIG. 2.

As an another example case of visual presentation in which a person who performs measurement can easily and intuitively figure out the situation, for example, as illustrated in FIG. 6, a graph of evaluation values (for example, a bar graph) that are associated with the subspaces that correspond to the subareas of the three-dimensionally measured object 30b may be displayed near the display area of the three-dimensionally measured object 30b (an example of a measured object). In the example as illustrated in FIG. 6, labels "1" to "4" are displayed in the subareas (i.e., the subareas 501 to 504) of the three-dimensionally measured object 30b, and the bar graphs of evaluation value that correspond to these labels are displayed. Note that the way of presentation where a person who performs measurement can easily and intuitively figure out the situation is not limited to a visual presentation, but may be, for example, an acoustic or vibrating presentation.

The way of presenting a graph of evaluation values is not limited to an example case as illustrated in FIG. 6, in which a display of the three-dimensionally measured object 30b and the graph of evaluation values are separately displayed in different display areas. Alternatively, the graph of an evaluation value that corresponds to the relevant subarea (or, for example, the evaluation value in number) may be superimposed on the display area of each subarea. Due to such a configuration, the relation between the subareas of the three-dimensionally measured object 30b and the evaluation values of the subspace that corresponds to that subarea can instantly be figured out without shifting the viewpoint between the three-dimensionally measured object 30b and the graph of evaluation values.

Moreover, the way of presenting a graph of evaluation values is not limited to a bar graph as illustrated in FIG. 6. For example, the bar graph may be changed to a different type of graph such as a radar chart.

As another example case of visual presentation in which a person who performs measurement can easily and intuitively figure out the situation, the evaluation values may be displayed in numbers.

As described above, the display control unit 207 may change the viewpoint of the three-dimensionally measured object to be displayed on the display unit 208, in accordance with the operation or input of changing the viewpoint of the three-dimensionally measured object of the object 30, which is accepted by the input unit 206. The display control unit 207 may change the viewpoint of the three-dimensionally measured object in accordance with the direction in which the three-dimensional measuring device 20 measures the object 30.

Various ways of display based on the evaluation values, as described above, are given as an example of displaying the information about the amount of the shape measurement data.

The display unit 208 is a functional unit that displays various kinds of data (for example, a three-dimensionally measured object represented by the shape measurement data) under the control of the display control unit 207. The display unit 208 may be implemented by the display 107 as illustrated in FIG. 2.

Note also that all of or some of the coordinate system setting unit 201, the space division unit 202, the acquisition unit 203, the determination unit 204, the evaluation value calculation unit 205, and the display control unit 207 may be implemented by hardware circuitry such as a field-programmable gate array (FPGA) or an application specific integrated circuit (ASIC), in place of a software program.

Note that the coordinate system setting unit 201, the space division unit 202, the acquisition unit 203, the determination unit 204, the evaluation value calculation unit 205, the input unit 206, the display control unit 207, and the display unit 208 merely indicate functions schematically, and no limitation is intended by such configurations. For example, a plurality of functional units that are illustrated as independent functional units in FIG. 3 may be configured as a single functional unit. Alternatively, the function of a single functional unit as illustrated in FIG. 3 may be divided into a plurality of functions implemented by a plurality of functional units.

As the number of divisions of three-dimensional coordinate space into subspaces, which is performed by the space division unit 202, is greater, the shape measurement data of what position and direction of the object 30 is insufficient can be displayed in a more detailed manner. However, if the three-dimensional coordinate space is divided into too many subspaces, the display of evaluation values becomes complicated. For this reason, the number of divisions into subspaces needs to be set to an optimal or desirable value.

### Processes Performed by Measurement Support Device

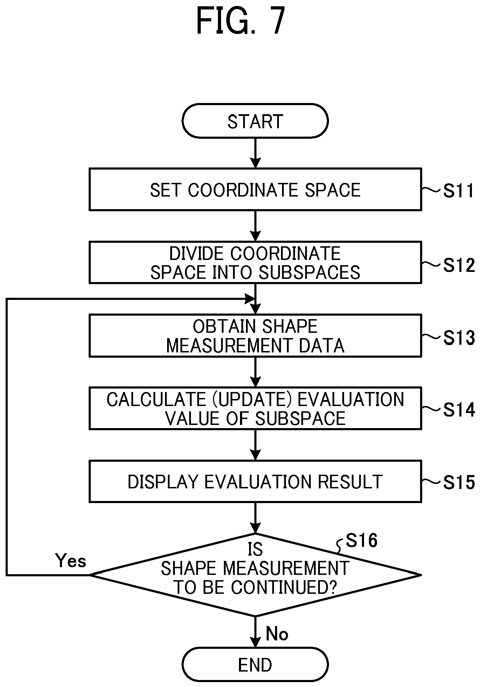

FIG. 7 is a flowchart of the processes performed by the measurement support device 10 according to the first embodiment of the present disclosure.

The processes that are performed by the measurement support device 10 according to the present embodiment are described below with reference to FIG. 7.

### Step S11

For example, the coordinate system setting unit 201 of the measurement support device 10 sets (constructs) the three-dimensional coordinate space based on a predetermined reference point. A person who performs measurement may control the measurement support device 10 to explicitly specify a reference point used to set three-dimensional coordinate space. When a rough shape of the object 30 is known, preliminarily set points may be used. Preferably, a characteristic point in the shape of the object 30 is used as a reference point. For example, when the object 30 is in a conical shape, the vertex or a point of intersection where a diameter of the base intersects the perimeter of a circle may be used as a reference point. Alternatively, when the object 30 is in a car, for example, the center of the front bumper or the rear bumper or edges of the right and left mirrors may be used as a reference point. If the object 30 is an asymmetrical object, the center of the object 30 or a point near the center of gravity may be used as a reference point. Then, the process shifts to the processes in a step S12.

### Step S12

The space division unit 202 of the measurement support device 10 divides the three-dimensional coordinate space set by the coordinate system setting unit 201 into a plurality of subspaces. Then, the process shifts to the processes in a step S13.

### Step S13

The acquisition unit 203 of the measurement support device 10 obtains the shape measurement data of the object 30 measured by the three-dimensional measuring device 20. Then, the process shifts to the processes in a step S14.

### Step S14

The determination unit 204 of the measurement support device 10 determines to which one of the subspaces of the three-dimensional coordinate space each part (or each area) of the three-dimensionally measured object represented by the shape measurement data (dot data) of the object 30 obtained by the acquisition unit 203 belongs. As described above, as the determination unit 204 determines to which one of the subspaces each part of the three-dimensionally measured object belongs, the three-dimensionally measured object is divided into a plurality of subareas.

Moreover, the evaluation value calculation unit 205 of the measurement support device 10 calculates (updates) the evaluation value of each subspace, based on the shape measurement data included in that subspace, i.e., the shape measurement data included in the subspace, which makes up the corresponding subarea of a three-dimensionally measured object. Then, the process shifts to the processes in a step S15.

### Step S15

The display control unit 207 of the measurement support device 10 causes the display unit 208 to display a three-dimensionally measured object represented by the shape measurement data obtained by the acquisition unit 203. In so doing, the display control unit 207 causes a display to display the evaluation result, i.e., the three-dimensionally measured object in visual presentation where a person who performs measurement can easily and intuitively figure out the situation, based on the evaluation value of the subspace that corresponds to each subarea of the three-dimensionally measured object. For example, the display control unit 207 based on the level of evaluation value of each subspace causes a display to display the subareas of the three-dimensionally measured object belonging to the multiple subspaces upon color-coding these subareas with different colors. Then, the process shifts to the processes in a step S16.

### Step S16

A person who performs measurement determines the positions of the object to be measured next and directions based on the visual presentation of evaluation values displayed by the display unit 208. When the person who performs the measurement determines that it is necessary to continue shape measurement ("YES" in the step S16), the process returns to the processes in the step S13, and the shape of the surface shape of the object 30 is measured using the three-dimensional measuring device 20. The acquisition unit 203 newly obtains the shape measurement data of the object 30 measured by the three-dimensional measuring device 20. As the processes in the steps S13 to S16 are repeated as described above, the evaluation values based on the amount of the shape measurement data included in the multiple subspace are updated, and the display of the corresponding subarea is updated based on the updated evaluation values.

By contrast, when the person who performs the measurement determines that the shape measurement is done ("NO" in the step S16), the shape measurement is terminated. The determination as to whether the shape measurement is to be terminated by the measurement support device 10 may automatically be performed by determining whether predetermined termination conditions (for example, when evaluation values equal to or greater than a predetermined value are obtained in all the subspaces) are satisfied. Alternatively, a message indicating that shape measurement data has been obtained to a sufficient degree may be displayed.

The processes that are performed by the measurement support device 10 according to the present embodiment are implemented by the processes as in the above steps S11 to S16.

As described above, the measurement support device 10 according to the present embodiment determines to which one of the subspaces a three-dimensionally measured object that is represented by the shape measurement data measured by the three-dimensional measuring device 20 belongs, and calculates and obtains the evaluation value for each one of the subspaces to which each one of the subareas belong. Then, the measurement support device 10 according to the present embodiment causes a display based on the obtained evaluation value to display the three-dimensionally measured object in visual presentation where a person who performs measurement can easily and intuitively figure out the situation. Due to such a configuration, a person who performs measurement (technician) can easily figure out whether the shape measurement data obtained by measuring the shape of a three-dimensional object is sufficient, and the person who performs the measurement (technician) can easily determine the positions of the object to be measured next and the directions. Even when the person who performs the measurement (technician) changes, the shape of a three-dimensional object can be measured homogenously.

### Modification

In a modification of the first embodiment of the present disclosure, operations in which the coordinate axes used to set (construct) the three-dimensional coordinate space are automatically determined based on the previously-obtained shape measurement data are described.

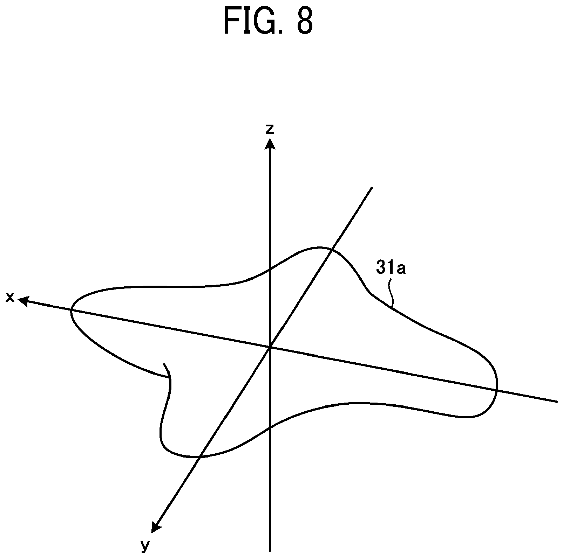

FIG. 8 is a diagram illustrating an example case in which a coordinate system is set based on the distribution of the shape measurement data, in the modification of the first embodiment of the present disclosure.

Operations are described with reference to FIG. 8, in which the coordinate axes used to set (construct) the three-dimensional coordinate space are automatically determined by the measurement support device 10 according to the modification of the second embodiment of the present disclosure, based on the previously-obtained shape measurement data. Note that the operations in the present modification of the first embodiment are explained based on the flowchart of FIG. 7 as described above.

### Step S11

Firstly, the coordinate system setting unit 201 of the measurement support device 10 sets (constructs) the initial three-dimensional coordinate space based on a predetermined reference point. As will be described later, the processes in the steps S11 to S16 are repeated based on the initial three-dimensional coordinate space until three-dimensional coordinate space is newly set by the coordinate system setting unit 201.

Further, when it is determined that the amount of shape measurement data (i.e., the number of points) obtained by the acquisition unit 203 has reached the amount required to automatically determine the coordinate axes of the three-dimensional coordinate space, the coordinate system setting unit 201 performs statistical processing such as main component analysis or independent component analysis on the previously-obtained shape measurement data, and extracts coordinate axes in which the distribution of the shape measurement data can be expressed in an efficient manner. As illustrated in FIG. 8, for example, the coordinate system setting unit 201 performs statistical processing on the distribution of the shape measurement data of a three-dimensionally measured object 31a (an example of a measured object) represented by the shape measurement data obtained by the acquisition unit 203, and extracts directions of distribution with large dispersion (i.e., the directions in which the groups of points of the shape measurement data distribute in an extended manner) as coordinate axes. In FIG. 8, directions of distribution in which the dispersion of the shape measurement data is large are extracted as the X-axis and Y-axis, and the direction orthogonal to both the X-axis and Y-axis is determined to be the Z-axis.

The determination as to whether the amount of obtained shape measurement data is sufficient to automatically determine the coordinate axes of the three-dimensional coordinate space may be performed based on a result of comparison of a prescribed threshold with a value such as the integrated value of the amount of shape measurement data (i.e., the number of points) or the value of the dispersion of the shape measurement data.

Further, the coordinate system setting unit 201 resets (reconstructs) the three-dimensional coordinate space based on the newly-extracted coordinate axis. After that, the reset three-dimensional coordinate space may continuously be used. When shape measurement data is newly obtained by a person who performs measurement, statistical processing may be performed again to extract new coordinate axes, and the three-dimensional coordinate space may be reset.

### Steps S12 to S15

The operations in steps S12 to S15 in the present modification of the first embodiment are equivalent to the operations as described above in the first embodiment of the present disclosure. Then, the process shifts to the processes in a step S16.

### Step S16

A person who performs measurement determines the positions of the object to be measured next and directions based on the visual presentation of evaluation values displayed by the display unit 208. When the person who performs the measurement determines that it is necessary to continue shape measurement ("YES" in the step S16), the process return to the processes in the step S11. As described above, when the three-dimensional coordinate space has already been reset in the step S11, the process may return to the processes in the step S13. On the other hand, when the person who performs the measurement determines that the shape measurement has sufficiently be done
("NO" in the step S16), the shape measurement is terminated.

The processes that are performed by the measurement support device 10 according to the present modification of the third embodiment of the present disclosure are implemented by the processes as in the above steps S11 to S16.

As described above, in the measurement support device 10 according to the present modification of the first embodiment of the present disclosure, it is no longer necessary for a person who performs measurement (a technician) to manually determine the coordinate axes for the three-dimensional coordinate space, and consequently the work load can be reduced.

When the validity of the subspace is poor and the reliability of the evaluation value cannot be guaranteed at a stage where the initial three-dimensional coordinate space is used in the step S11, preferably the subspace or the evaluation value should not be displayed by the display unit 208. By contrast, preferably, the shape measurement data obtained by the acquisition unit 203 is displayed on an as-needed basis, because the person who performs the measurement can determine the positions to be measured next and directions in view of the displayed shape measurement data.

### Second Embodiment

Differences between the measurement support device 10 according to a second embodiment of the present disclosure and the measurement support device 10 according to the first embodiment of the present disclosure are described below.

In the description of the first embodiment of the present disclosure, an object to be measured was not limited in particular. By contrast, in the present embodiment, operations in which the shape of a human head is measured as an object are described. For example, it is expected that the three-dimensionally measured object obtained by measures the shape of a human head be applied to a healthcare field (e.g., a field of magneto-encephalography). An overall configuration of the measurement support system 1 according to the present embodiment and a hardware configuration and a configuration of the functional blocks of the measurement support device 10 are equivalent to the configurations as described above in the first embodiment of the present disclosure. In the present embodiment, cases in which a digitizer is used as the three-dimensional measuring device 20 are described.

### Operations of Functional Blocks of Measurement Support Device

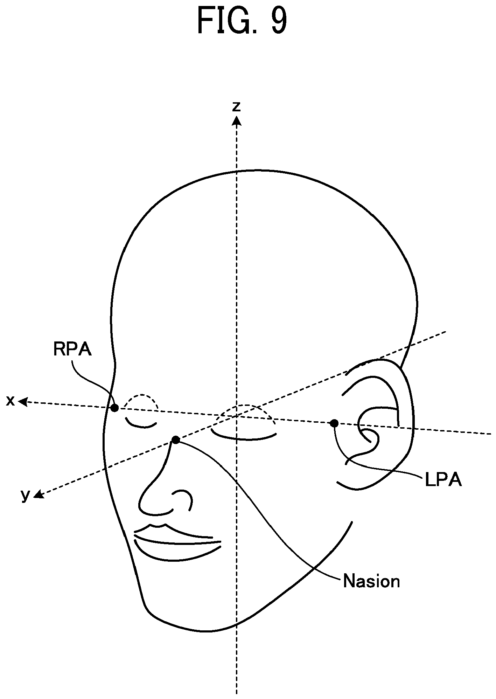

The operations of functional blocks of the measurement support device 10 according to the present embodiment are described below with reference to FIG. 9 to FIG. 11.

FIG. 9 is a diagram illustrating a method of setting a reference point of a head according to the second embodiment of the present disclosure.

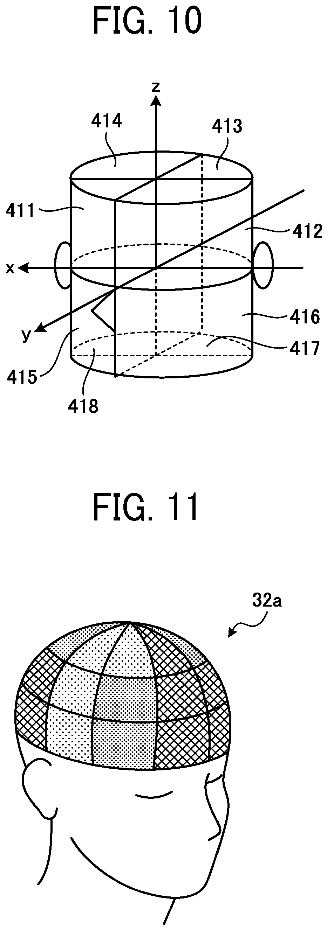

FIG. 10 is a diagram illustrating an example case in which coordinate space is divided into a plurality of subspaces, according to the second embodiment of the present disclosure.

FIG. 11 is a three-dimensionally measured object, which corresponds to the object 30 in the above embodiment, is color-coded on a subarea-by-subarea basis, according to the second embodiment of the present disclosure.

Firstly, a person who performs measurement uses a digitizer that serves as the three-dimensional measuring device 20 (such a three-dimensional measuring device may be referred to as a digitizer in the following description of the present embodiment) to specify a reference point of the three-dimensional coordinate space in a human head. For example, a characteristic bump or dip in the skull, which is referred to as an anatomic reference point, may be used as a reference point. For example, the anatomic reference point may be, for example, a nasion, a left pre-auricular point (LPA), a right pre-auricular point (RPA), and an external occipital protuberance (inion). In the present embodiment, a nasion, a left pre-auricular point (LPA), and a right pre-auricular point (RPA) are used as reference points. In other words, a person who performs measurement uses a digitizer (i.e., the three-dimensional measuring device 20) to specify the positions of the nasion, LPA, and RPA of a human head, which is an object to be measured, as positions that serve as the reference points of the three-dimensional coordinate space, and the acquisition unit 203 obtains the shape measurement data of each position.

As illustrated in FIG. 9, the coordinate system setting unit 201 sets the straight line drawn from the LPA to the RPA to be the X-axis. Moreover, the coordinate system setting unit 201 sets the straight line orthogonal to the X-axis and drawn from that orthogonal point to the nasion to be the Y-axis. Moreover, the coordinate system setting unit 201 sets the straight line orthogonal to the X-axis and the Y-axis and drawn from that orthogonal point towards the parietal direction to be the Z-axis. Further, the coordinate system setting unit 201 sets (constructs) the three-dimensional coordinate space that is defined by the X-axis, Y-axis, and the Z-axis as obtained above.

The space division unit 202 divides the three-dimensional coordinate space set by the coordinate system setting unit 201 into a plurality of subspaces. As illustrated in FIG. 10, for example, the space division unit 202 separates the three-dimensional coordinate space set by the coordinate system setting unit 201 by each of the xy-plane, the yz-plane, and zx-plane to form eight subspaces (i.e., subspaces 411 to 418).

Division into subspaces as illustrated in FIG. 10 is given by way of example. For example, the zx-plane may be rotated around the Z-axis to divide the three-dimensional coordinate space with an angle smaller than 90 degrees in the direction perpendicular to the xy-plane. Alternatively, for example, when the three-dimensional coordinate space is divided for every 45 degrees, the three-dimensional coordinate space is divided into sixteen subspaces.

The acquisition unit 203 obtains the shape measurement data of the object 30 measured by a digitizer (i.e., the three-dimensional measuring device 20). Then, the acquisition unit 203 stores the obtained shape measurement data in the auxiliary memory 104. As a method of measures the shape of an object using a digitizer, for example, the shape measurement data of points may be obtained by specifying a point on a one-by-one basis, or the shape measurement data of groups of points may be obtained by sliding a digitizer along the surface shape of a head.

When the obtained shape measurement data consists of dots, the subspaces are uniquely determined. However, when the obtained shape measurement data consists of line segments, a plurality of subspaces may overlap with each other. In such a configuration, the line segments may be decomposed into several dot components, and each dot may be assigned to the subspaces. Alternatively, the center of each line segment is calculated, and the entirety of each line segment may be assigned to the subspace to which the calculated center point belongs.

The operations that are performed by the determination unit 204, the evaluation value calculation unit 205, the input unit 206, and the display unit 208 are equivalent to the operations as described above in the first embodiment of the present disclosure.

The display control unit 207 causes the display operation of the display unit 208. More specifically, the display control unit 207 causes the display unit 208 to display a three-dimensionally measured object represented by the shape measurement data obtained by the acquisition unit 203. In so doing, the display control unit 207 causes a display to display the three-dimensionally measured object in visual presentation where a person who performs measurement can easily and intuitively figure out the situation, based on the evaluation value of the subspace that corresponds to each subarea of the three-dimensionally measured object. As illustrated in FIG. 10, for example, the display control unit 207 causes a display to display the subareas of the three-dimensionally measured object belonging to the multiple subspaces upon color-coding these subareas with different colors, based on the level of evaluation value of each of the eight subspaces as divided above. FIG. 11 illustrates a three-dimensionally measured object 32a of a human head (an example of a measured object). More specifically, FIG. 11 illustrates a case in which three-dimensional coordinate space is divided into a larger number of subspaces than the subspaces as illustrated in FIG. 10, and illustrates an example case in which each subarea that belongs to one of the multiple subspaces is displayed upon color-coding these subareas with different colors according to the corresponding evaluation value.

Note that the processes that are performed by the measurement support device 10 according to the present embodiment are equivalent to the flow of processes in the first embodiment of the present disclosure as described above with reference to FIG. 7.

As described above, when the measurement support device 10 according to the present embodiment measures the shape of a human head that serves as an object to be measured, three-dimensional coordinate space is constructed using anatomic reference points as reference points. Due to such a configuration, the association between the attitude of a human head and the displayed distribution of the shape measurement data and the evaluation values can easily be figured out.

### Modification

In a modification of the second embodiment of the present disclosure, operations are described in which a three-dimensionally measured object is two-dimensionally projected and displayed when evaluation values are to be displayed.

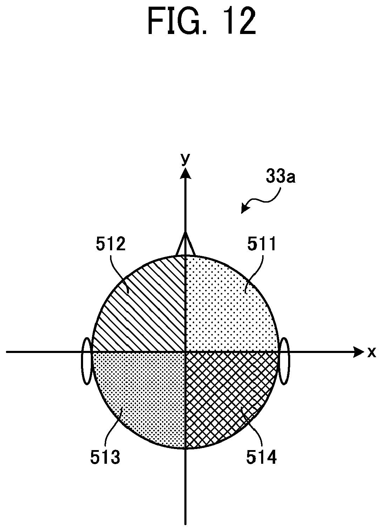

FIG. 12 is a diagram illustrating an example case in which the two-dimensionally projected image of a measured object 33a of the object 30 are color-coded, in the modification of the second embodiment of the present disclosure. operations in which the measurement support device 10 according to the modification of the second embodiment of the present disclosure two-dimensionally projects and displays a three-dimensionally measured object when evaluation values are to be displayed are described with reference to FIG. 12.

For example, the coordinate system setting unit 201 sets (constructs) the coordinate space (i.e., a two-dimensional plane in actuality) obtained by projecting the three-dimensional coordinates as described above in the second embodiment of the present disclosure onto any desired one of the planes. As illustrated in FIG. 12, for example, the coordinate system setting unit 201 sets (constructs) the coordinate space (two-dimensional plane) that is obtained by projecting three-dimensional coordinate space onto an xy-plane. In other words, when the coordinate space is set by the coordinate system setting unit 201, only the two axes (i.e., the X-axis and Y-axis in the example case as illustrated in FIG. 12) may be used from among the three-dimensional coordinate axes.

The space division unit 202 divides the coordinate space (xy-plane) set by the coordinate system setting unit 201 into a plurality of subspaces. For example, in the example case as illustrated in FIG. 12, the space division unit 202 divides the coordinate space (xy-plane) set by the coordinate system setting unit 201 into four subspaces including the first to fourth quadrants. Note that the term "space" in the terms "coordinate space" and "subspace" indicates not only three-dimensional space but also indicates a two-dimensional plane.

Then, the acquisition unit 203 obtains the shape measurement data of the object 30 measured by the three-dimensional measuring device 20. Then, the acquisition unit 203 stores the obtained shape measurement data in the auxiliary memory 104. In so doing, the display control unit 207 projects the three-dimensionally measured object represented by the shape measurement data obtained by the acquisition unit 203 onto the two-dimensional plane constructed by the coordinate system setting unit 201, and causes the display unit 208 to display the projected image as a two-dimensionally measured object (for example, the measured object 33a as illustrated in FIG. 12).

The determination unit 204 determines to which one of the subspaces of the coordinate space set by the coordinate system setting unit 201 each part (or each area) of the object represented by the shape measurement data (dot data) of the object obtained by the acquisition unit 203 belongs. In other words, the measured object is divided into a plurality of subareas as the determination unit 204 determines to which one of the subspaces each part of the measured object belongs.

By way of example, an object 33a that is represented by the shape measurement data of the object obtained by the acquisition unit 203 is illustrated in FIG. 12. As described above, the coordinate space (two-dimensional plane) set by the coordinate system setting unit 201 is divided into four subspaces including the first to fourth quadrants in the present modification of the second embodiment, and the measured object 33a is included in the subspaces composed of the first to fourth quadrants. In the object 33a, the determination unit 204 determines that the subarea 511 belongs to a first quadrant, and determines that the subarea 512 belongs to a second quadrant. Moreover, the determination unit 204 determines that the subarea 513 belongs to a third quadrant, and determines that the subarea 514 belongs to a fourth quadrant.

The evaluation value calculation unit 205 calculates the evaluation value of each subspace, based on the shape measurement data included in that subspace, i.e., the shape measurement data included in the subspace, which makes up the corresponding subarea of a measured object. The evaluation values that are calculated in the present modification of the second embodiments are similar to those in the first embodiment of the present disclosure as described above.

As described above, the display control unit 207 projects the three-dimensionally measured object represented by the shape measurement data obtained by the acquisition unit 203 onto the two-dimensional plane constructed by the coordinate system setting unit 201, and causes the display unit 208 to display the projected image as a two-dimensionally measured object. In so doing, the display control unit 207 controls a display to display the three-dimensionally measured object in visual presentation where a person who performs measurement can easily and intuitively figure out the situation, based on the evaluation value of the subspace that corresponds to each subarea of the measured object. As illustrated in FIG. 12, for example, the display control unit 207 displays the subareas of the measured object 33a (i.e., the subareas 511 to 514 in FIG. 12) belonging to the multiple subspaces upon color-coding these subareas with different colors based on the level of evaluation value of each subspace. A way of color-coding is similar to the way in the first embodiments of the present disclosure as described above.

As an another example case of visual presentation in which a person who performs measurement can easily and intuitively figure out the situation, for example, as described above in the description of the first embodiments of the present disclosure, a graph of evaluation values (for example, a bar graph) that are associated with the subspaces that correspond to the subareas of the measured object may be displayed near the display area of the measured object.

As described above, when the measurement support device 10 according to the present modification of the second embodiment of the present disclosure measures the shape of a human head that serves as an object to be measured, a three-dimensionally measured object represented by the obtained shape measurement data is projected onto the two-dimensional plane constructed by the coordinate system setting unit 201, and the projected image is displayed as a two-dimensionally measured object. Further, a display is controlled to display the object in visual presentation where a person who performs measurement can easily and intuitively figure out the situation, based on the evaluation value of each subspace. Due to such a configuration, assessment of the situation of the acquisition of the shape measurement data of the object 30 becomes easier by decreasing by one the number of dimensionalities of the information to be figured out by a person who performs measurement.

### Third Embodiment

In regard to a measurement support device 10a according to a third embodiment, differences from the measurement support device 10 according to the first embodiment of the present disclosure are described. An overall configuration of the measurement support system 1 according to the present embodiment and a hardware configuration of the measurement support device 10a are equivalent to the configurations as described above in the first embodiment of the present disclosure.

In the first embodiment of the present disclosure, noise that may contaminate the shape measurement data of a measured object is not taken into consideration. In actuality, the shape measurement data measured by the three-dimensional measuring device 20 may be contaminated with noise due to factors described below. In the present embodiment, processes are performed after the shape measurement data contaminated with noise is removed.

### Admixture of Noise

When the three-dimensional measuring device 20 is used to measure the surface shape of the object, unnecessary shape measurement data may be obtained due to the first to third factors as given below.

### First Factor

Noise due to the capability or performance of the three-dimensional measuring device 20

### Second Factor

Reflection or glare of an object other than the object to be measured

### Third Factor

Reflection or glare of a part of the object to be measured but not an area of interest
The shape measurement data that involves the effects caused by the above first to third factors is collectively referred to as "noise" in the following description. If the shape measurement data that is measured by the three-dimensional measuring device 20 is used just as it is to calculate the evaluation value and evaluate the sufficiency, the evaluation values and the sufficiency may not represent the realities appropriately when there was an admixture of noise as above. This situation stands out when the proportion of the amount of noise that occupies the shape measurement data is large. In order to handle such a situation, in the present embodiment, operations in which an evaluation value is calculated upon removing noise from the shape measurement data are described.

For example, noise may be removed using the following first to third methods.

In a first method, a statistical method may be used. In such a method, when the three-dimensional measuring device 20 is used to measure the shape measurement data, smoothing is spatially performed using, for example, an average filter or a median filter, based on an assumption that continuous shape measurement data is spatially continuous in a smooth manner. The filter may be exerted one-dimensionally, two-dimensionally, or three-dimensionally. This method can easily be implemented as its processes are relatively simple, and this method is effective in reducing discrete noise. On the other hand, this method is not very effective in reducing continuously existing noise (for example, reflection or glare of another object).

In a second method, the three-dimensional geometric configuration of an object may be used. In the second method, the space in which shape measurement data could exist is limited in view of the knowledge of the geometric configuration of a three-dimensional object, which is obtained beforehand. For example, the point of origin of the three-dimensional coordinate system or the distance between the center of gravity and the previously-obtained shape measurement data group may be used. When the shape of an object to be measured is approximately known, a three-dimensional model may be generated based on the known information, and the shape of the generated three-dimensional model may be corrected based on the obtained data. As a limitation, the inside of the three-dimensional model as obtained above may be used as space in which the shape measurement data could exist. Such a method is advantageous when the object to be measured is spatially apart from the source of noise, as in, for example, reflection or glare of another object. On the other hand, it is difficult to apply such a method to noise existing near the object.

In a third method, the geometric configuration of the surface of an object may be used. In the third method, the relative positions of the continuous shape measurement data is restricted in view of the knowledge of the geometric configuration of the surfaces of an object, which is obtained beforehand. For example, when the object is shaped like a sphere, there is little likelihood that a displacement vector of the continuous shape measurement data is parallel to the direction of a radius vector, and it can be considered that the shape measurement data producing a displacement vector that is nearly parallel to the direction of a radius vector involves noise. In such a method, noise existing near the object can be removed. In the present embodiment, noise reduction processes making use of the above third method are described.

### Operations and Configuration of Functional Blocks of Measurement Support Device

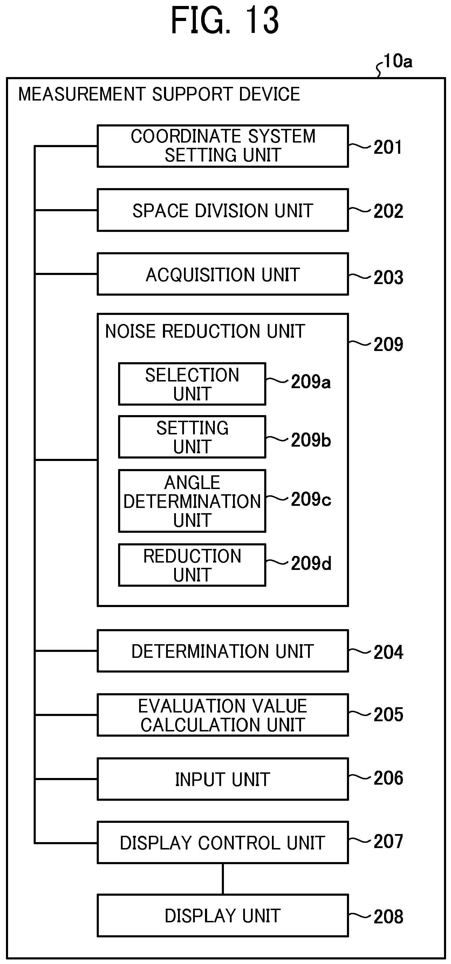

FIG. 13 is a diagram illustrating a configuration of functional blocks of a measurement support device 10a according to the third embodiment of the present disclosure.

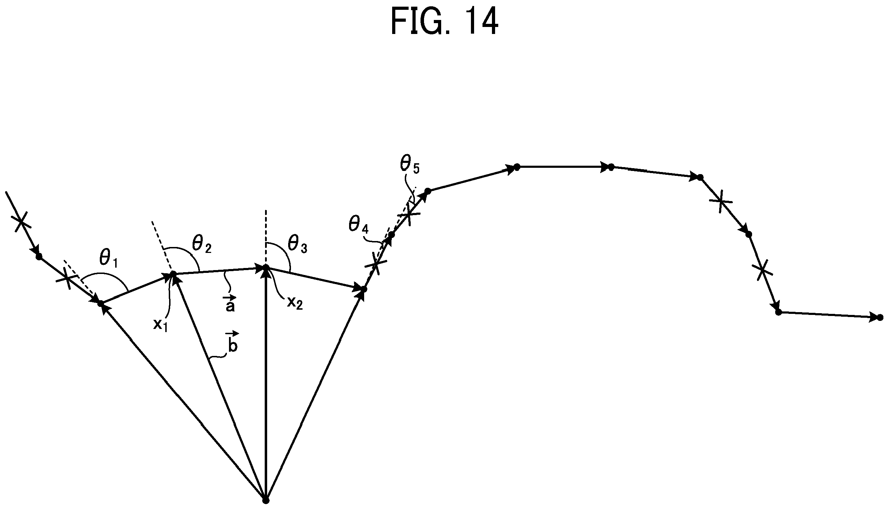

FIG. 14 is a diagram illustrating how vectors are set and how noise determination processes are performed in the third embodiment of the present disclosure.

Operations and a configuration of functional blocks of the measurement support device 10a according to the present embodiment are described below with reference to FIG. 13 and FIG. 14.

As illustrated in FIG. 13, the measurement support device 10a includes the coordinate system setting unit 201, the space division unit 202, the acquisition unit 203, the noise reduction unit 209, the determination unit 204, the evaluation value calculation unit 205, the input unit 206, the display control unit 207, and the display unit 208. Note that the processes in the coordinate system setting unit 201, the space division unit 202, the acquisition unit 203, the evaluation value calculation unit 205, the input unit 206, the display control unit 207, and the display unit 208 are equivalent to the operations as described above in the first embodiment of the present disclosure.

The noise reduction unit 209 is a functional unit that removes some of the shape measurement data that is determined to be noise in the shape measurement data (dot data) of the object 30 obtained by the acquisition unit 203. As illustrated in FIG. 13, the noise reduction unit 209 includes a selection unit 209a, a setting unit 209b, an angle determination unit 209c, and a removal unit 209d.

The selection unit 209a is a functional unit that selects any desired two items of shape measurement data that are temporally or spatially close to each other, from among a plurality of items of the shape measurement data obtained by the acquisition unit 203. The expression "temporally close" indicates that the time measured by the three-dimensional measuring device 20 is close. The expression "spatially close" indicates that the distance is short. As illustrated in FIG. 14, for example, the selection unit 209a selects points x1 and x2 (shape measurement data) that are temporally or spatially close to each other, from among several items of shape measurement data.

The setting unit 209b is a functional unit that sets the displacement vector formed by two points of the shape measurement data and the vector that is determined by the point of origin (for example, the center point when the object 30 has a spherical shape) (an example of a predetermined point) and one of the two points that form the displacement vector (such a vector may be referred to as a position vector in the following description). As illustrated in FIG. 14, for example, the setting unit 209b sets a displacement vector a determined by points x₁ and x₂ of the shape measurement data (the vector a is indicated with a with arrow on the top in FIG. 14, which is an appropriate expression for vectors) and a position vector b determined by the point x₁ and the point of origin (the vector b is indicated with b with arrow on the top in FIG. 14).

In regard to the selected two points of the shape measurement data that form a displacement vector, the direction in which one of these two points heads for the other point may be any one of the two directions. A position vector may be a vector that heads for one of the two points of the shape measurement data from the point of origin, or may be a vector with the opposite direction.

The angle determination unit 209c is a functional unit that determines whether the angle θ that the two vectors (i.e., the displacement vector and the position vector) set by the setting unit 209b form satisfies a predetermined condition. In the example as illustrated in FIG. 14, angles *θ*₁ to *θ*₅ are illustrated as the angles formed by displacement vectors and position vectors. In this configuration, the angle θ that the two vectors (i.e., the displacement vector a and the position vector b) form is calculated and obtained using a first equation given below where the inner products of the vectors are used.

### First Equation

In the shape measurement data obtained by the acquisition unit 203, preferably, the angle θ that the displacement vector and the position vector form is close to 90 degrees (right angle). For this reason, a predetermined condition is, for example, is a condition as to whether |*θ*-90| exceeds a predetermined threshold. As illustrated in FIG. 14, for example, the angle determination unit 209c determines whether the |*θ*₂-90| exceeds a predetermined threshold, where *θ*₂ denotes the angle that the displacement vector a and the position vector b at a point x₁ form.

When it is determined by the angle determination unit 209c that a predetermined condition is not satisfied, the removal unit 209d is a functional unit that determines that at least one of the two items of shape measurement data selected by the selection unit 209a involves noise and removes the shape measurement data as noise. As illustrated in FIG. 14, for example, the displacement vectors marked with crosses "x" indicate that the angle formed with the corresponding position vector does not satisfy the above predetermined condition, and the shape measurement data (points) that correspond to those displacement vectors indicate the shape measurement data that is determined to involve noise. In the present embodiment, the removal unit 209d may remove the shape measurement data that is determined by the angle determination unit 209c not to satisfy a predetermined condition and the shape measurement data subsequent thereto.

For example, the noise reduction unit 209 may be implemented by a program executed by the CPU 101 as illustrated in FIG. 2.

Note also that the noise reduction unit 209 may reduce or remove noise every time the acquisition unit 203 obtains shape measurement data, or may reduce or remove noise after a predetermined amount of the obtained shape measurement data is accumulated.

Note also that all of or some of the coordinate system setting unit 201, the space division unit 202, the acquisition unit 203, the noise reduction unit 209, the determination unit 204, the evaluation value calculation unit 205, and the display control unit 207 may be implemented by hardware circuitry such as a field-programmable gate array (FPGA) or an application specific integrated circuit (ASIC), in place of a software program.

Moreover, the coordinate system setting unit 201, the space division unit 202, the acquisition unit 203, the noise reduction unit 209, the determination unit 204, the evaluation value calculation unit 205, the input unit 206, the display control unit 207, and the display unit 208 merely indicate functions schematically, and no limitation is intended by such configurations. For example, a plurality of functional units that are illustrated as independent functional units in FIG. 13 may be configured as a single functional unit. Alternatively, a function of a single functional unit as illustrated in FIG. 13 may be divided into a plurality of functions implemented by a plurality of functional units.

### Processes Performed by Measurement Support Device

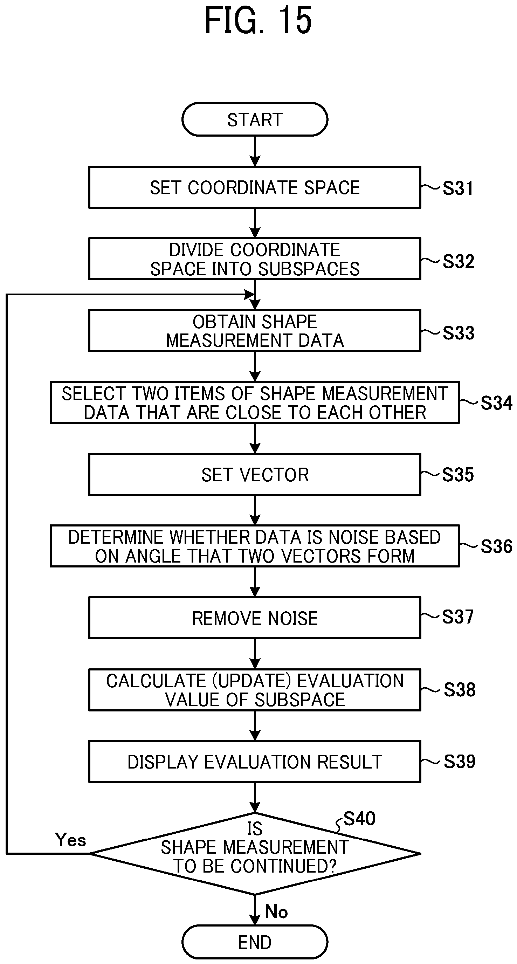

FIG. 15 is a flowchart of the processes performed by the measurement support device 10a according to the third embodiment of the present disclosure.

The processes that are performed by the measurement support device 10a according to the present embodiment is described below with reference to FIG. 15.

### Steps S31 to S33

The processes in steps S31 to S33 are equivalent to the processes in the steps S11 to S13 as described above with reference to FIG. 7, respectively. After the processes in the step S33 end, the process shifts to the processes in a step S34.

### Step S34

The selection unit 209a of the noise reduction unit 209 of the measurement support device 10a selects any desired two items of shape measurement data that are temporally or spatially close to each other, from among a plurality of items of the shape measurement data obtained by the acquisition unit 203. Then, the process shifts to the processes in a step S35.

### Step S35

The setting unit 209b of the noise reduction unit 209 of the measurement support device 10a sets the displacement vector formed by two points of the shape measurement data and the position vector determined by the point of origin and one of the two points that form the displacement vector. Then, the process shifts to the processes in a step S36.

### Step S36

The angle determination unit 209c of the noise reduction unit 209 of the measurement support device 10a determines whether the angle θ that the two vectors (i.e., the displacement vector and the position vector) set by the setting unit 209b form satisfies a predetermined condition. For example, the angle determination unit 209c determines whether |*θ*-90| exceeds a predetermined threshold, where *θ* denotes the angle that these two vectors form. Then, the process shifts to the processes in a step S37.

### Step S37

When it is determined by the angle determination unit 209c that a predetermined condition is not satisfied, the removal unit 209d of the noise reduction unit 209 of the measurement support device 10a determines that at least one of the two items of shape measurement data selected by the selection unit 209a involves noise, and removes the shape measurement data as noise. After all the shape measurement data obtained by the acquisition unit 203 goes through the processes in the steps S34 to S37, the process shifts to the processes in a step S38.

### Steps S38 to S40

The processes in steps S38 to S40 are equivalent to the processes in the steps S14 to S16 as described above with reference to FIG. 7, respectively.

The processes that are performed by the measurement support device 10a according to the present embodiment are implemented by the processes as in the above steps S31 to S40.

As described above, the measurement support device 10a according to the present embodiment removes the shape measurement data that is determined to involve noise from the shape measurement data measured by the three-dimensional measuring device 20, based on the angle that the displacement vector and the position vector form, to remove unnecessary groups of points that do not belong to the object to be measured. Due to such a configuration, noise existing near the object can be removed, and the evaluation value can be calculated and obtained based on valid groups of points (consistent with the purposes of a person who performs measurement) extracted from the shape measurement data measured by the three-dimensional measuring device 20. Accordingly, the progress of shape measurement can appropriately be quantified, and the person who performs the measurement (technician) can easily figure out whether the shape measurement data obtained by measuring the shape of an object is sufficient.

### First Modification

For example, when the surface shape of an object is smooth, the angle that two displacement vectors of the continuous shape measurement data form it is expected be small. In the present modification of the third embodiment of the present disclosure, as noise reduction processes in which the above third method is adopted, operations in which whether noise is involved is determined based on the angle that the two displacement vectors of the continuous shape measurement data form are described.

### Operations of Functional Blocks of Measurement Support Device

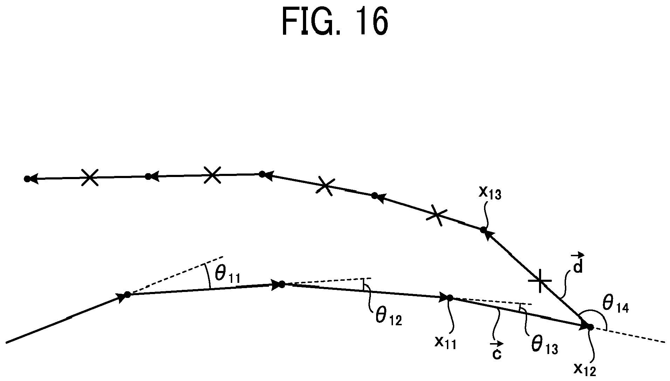

FIG. 16 is a diagram illustrating how vectors are set and how noise determination processes are performed in the first modification of the third embodiment of the present disclosure.

The operations of functional blocks of the measurement support device 10a according to the present embodiment are described with reference to FIG. 16. Note also that the configuration of functional blocks of the measurement support device 10a according to the present modification is equivalent to the configuration as described above with reference to FIG. 13. Among the functional blocks as illustrated in FIG. 13, the operations that are different from the operations of the functional blocks of the measurement support device 10a according to the third embodiment as described above are described.

In a similar manner to the configuration as illustrated in FIG. 13, the noise reduction unit 209 according to the present modification of the third embodiment of the present disclosure includes the selection unit 209a, the setting unit 209b, the angle determination unit 209c, and the removal unit 209d.

The selection unit 209a selects any desired three items of shape measurement data that are temporally or spatially close to each other, from among a plurality of items of the shape measurement data obtained by the acquisition unit 203. For example, as illustrated in FIG. 16, the selection unit 209a selects points x11 to x13 (shape measurement data) that are temporally or spatially close to each other, from among several items of shape measurement data.

The setting unit 209b selects any desired two pairs of shape measurement data from among three points of shape measurement data, and sets the two displacement vectors that are determined by these pairs of points of shape measurement data. For example, as illustrated in FIG. 16, the setting unit 209b sets a displacement vector c drawn from the point x₁₁ to the point x₁₂ (the vector c is indicated with c with arrow on the top in FIG. 16, which is an appropriate expression for vectors) and a displacement vector d drawn from the point x₁₂ to the point x₁₃ (the vector d is indicated with d with arrow on the top in FIG. 16). Note that these points x₁₁, x₁₂, and x₁₃ are components of the shape measurement data.

In regard to the selected two points of the shape measurement data that form a displacement vector, the direction in which one of these two points heads for the other point may be any one of the two directions. The displacement vectors that are to be set based on the selected points x₁₁ to x₁₃ as illustrated in FIG. 16 may be, for example, a displacement vector drawn from the point x₁₁ to the point x₁₂ and a displacement vector drawn from the point x x₁₁ to the point x₁₃. Alternatively, a displacement vector drawn from the point x₁₁ to the point x₁₃ and a displacement vector drawn from the point x₁₂ to the point x₁₃ may be set.

The angle determination unit 209c determines whether the angle θ that the two displacement vectors set by the setting unit 209b form satisfies a predetermined condition. In the example as illustrated in FIG. 16, angles *θ*₁₁ to *θ*₁₄ are illustrated as the angles that the two displacement vectors form. In this configuration, the angle θ that the two displacement vectors form is calculated and obtained using the first equation as described above.

In the shape measurement data obtained by the acquisition unit 203, preferably, the angle θ that two displacement vectors form is close to 0 degree or 180 degrees (parallel). For this reason, a predetermined condition is based on, for example, whether |sin *θ*| exceeds a predetermined threshold. As illustrated in FIG. 16, for example, the angle determination unit 209c determines whether |sin *θ*₁₄| exceeds a predetermined threshold, where *θ*₁₄ denotes the angle that the displacement vector c and the displacement vector d form.

When it is determined by the angle determination unit 209c that a predetermined condition is not satisfied, the removal unit 209d determines that at least one of the three items of shape measurement data selected by the selection unit 209a involves noise, and removes the shape measurement data as noise. The removal unit 209d may remove the shape measurement data that is determined by the angle determination unit 209c not to satisfy a predetermined condition and the shape measurement data subsequent thereto. For example, as illustrated in FIG. 16, the displacement vectors marked with crosses "x" indicate that the angle formed with the corresponding position vector does not satisfy the above predetermined condition, and the shape measurement data (points) that correspond to those displacement vectors indicates the displacement vector that is determined to involve noise and the displacement vector composed of the shape measurement data that corresponds to that displacement vector and the shape measurement data subsequent thereto.

### Processes Performed by Measurement Support Device

The processes that are performed by the measurement support device 10a according to the present modification of the third embodiment of the present disclosure is described with reference to the above flowchart as illustrated in FIG. 15.

### Steps S31 to S33

The processes in steps S31 to S33 are equivalent to the processes in the steps S11 to S13 as described above with reference to FIG. 7, respectively. After the processes in the step S33 end, the process shifts to the processes in a step S34.

### Step S34

The selection unit 209a of the noise reduction unit 209 of the measurement support device 10a selects any desired three items of shape measurement data that are temporally or spatially close to each other, from among a plurality of items of the shape measurement data obtained by the acquisition unit 203. Then, the process shifts to the processes in a step S35.

### Step S35

The setting unit 209b of the noise reduction unit 209 of the measurement support device 10a selects any desired two pairs of shape measurement data from among three points of shape measurement data, and sets the two displacement vectors that are determined by these pairs of points of shape measurement data. Then, the process shifts to the processes in a step S36.

### Step S36

The angle determination unit 209c of the noise reduction unit 209 of the measurement support device 10a determines whether the angle θ that the two displacement vectors set by the setting unit 209b form satisfies a predetermined condition. For example, the angle determination unit 209c determines whether |sin *θ*| exceeds a predetermined threshold, where *θ* denotes the angle that these two vectors form. Then, the process shifts to the processes in a step S37.

### Step S37

When it is determined by the angle determination unit 209c that a predetermined condition is not satisfied, the removal unit 209d of the noise reduction unit 209 of the measurement support device 10a determines that at least one of the three items of shape measurement data selected by the selection unit 209a involves noise, and removes the shape measurement data as noise. After all the shape measurement data obtained by the acquisition unit 203 goes through the processes in the steps S34 to S37, the process shifts to the processes in a step S38.

### Steps S38 to S40

The processes in steps S38 to S40 are equivalent to the processes in the steps S14 to S16 as described above with reference to FIG. 7, respectively.

The processes that are performed by the measurement support device 10a according to the present modification of the third embodiment of the present disclosure are implemented by the processes as in the above steps S31 to S40.

As described above, the measurement support device 10b according to the present modification of the third embodiment of the present disclosure removes the shape measurement data that is determined to involve noise from the shape measurement data measured by the three-dimensional measuring device 20, based on the angle that the two displacement vectors form, to remove unnecessary groups of points that do not belong to the object to be measured. Due to such a configuration, noise existing near the object can be removed, and the evaluation value can be calculated and obtained based on valid groups of points (consistent with the purposes of a person who performs measurement) extracted from the shape measurement data measured by the three-dimensional measuring device 20. Accordingly, the progress of shape measurement can appropriately be quantified, and the person who performs the measurement (technician) can easily figure out whether the shape measurement data obtained by measuring the shape of an object is sufficient.

### Second Modification

For example, when the object is shaped like a sphere, it is expected that a difference between the length of one position vector (i.e., the distance between the point of origin and a specific point of the shape measurement data) and the length of another position vector be small. In the second modification of the third embodiment of the present disclosure, as noise reduction processes in which the above third method is adopted, operations in which whether noise is involved is determined based on the length of a position vector of the shape measurement data are described.

Operations and Configuration of Functional Blocks of Measurement Support Device

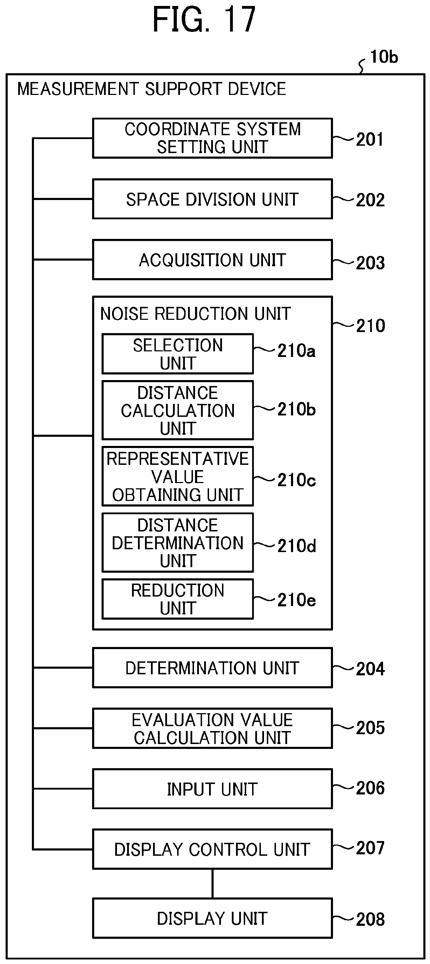

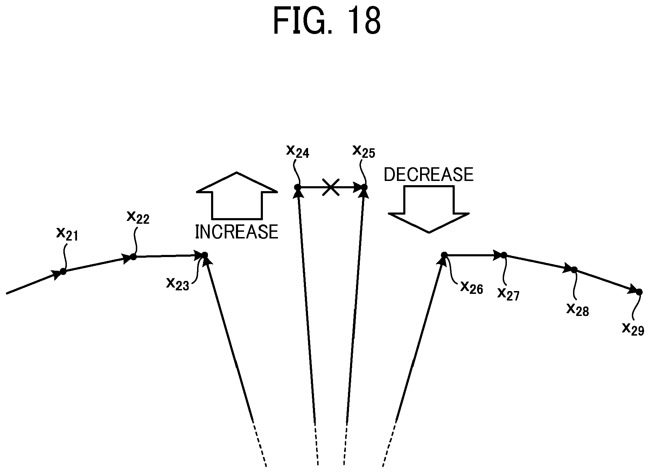

The operations and the configuration of functional blocks of the measurement support device 10b according to the present modification of the third embodiment of the present disclosure are described with reference to FIG. 17 and FIG. 18.

FIG. 17 is a diagram illustrating a configuration of the functional blocks of a measurement support device 10b according to a second modification of the third embodiment of the present disclosure.

FIG. 18 is a diagram illustrating how vectors are set and how noise determination processes are performed in the second modification of the third embodiment of the present disclosure.

As illustrated in FIG. 17, the measurement support device 10b includes the coordinate system setting unit 201, the space division unit 202, the acquisition unit 203, the noise reduction unit 210, the determination unit 204, the evaluation value calculation unit 205, the input unit 206, the display control unit 207, and the display unit 208. Note that the processes in the coordinate system setting unit 201, the space division unit 202, the acquisition unit 203, the evaluation value calculation unit 205, the input unit 206, the display control unit 207, and the display unit 208 are equivalent to the operations as described above in the first embodiment of the present disclosure.

The noise reduction unit 210 is a functional unit that removes some of the shape measurement data that is determined to be noise in the shape measurement data (dot data) of the object 30 obtained by the acquisition unit 203. As illustrated in FIG. 17, the noise reduction unit 210 includes a selection unit 210a, a distance calculation unit 210b, a representative value obtaining unit 210c, a distance determination unit 210d, and a removal unit 210e.

The selection unit 210a is a functional unit that selects any desired multiple items of shape measurement data that are temporally or spatially close to each other, from among a plurality of items of the shape measurement data obtained by the acquisition unit 203. For example, as illustrated in FIG. 18, the selection unit 210a selects points x21 to x29 (multiple items of shape measurement data) that are temporally or spatially close to each other, from among several items of shape measurement data.

The distance calculation unit 210b is a functional unit that calculates the distance (the length of a position vector at each point) between each of the points of the shape measurement data selected by the selection unit 210a and the point of origin (for example, the center point when the object 30 has a spherical shape) (an example of a predetermined point). For example, as illustrated in FIG. 18, the distance calculation unit 210b calculates the distance between the point of origin and each one of the points x₂₁ to x₂₉, i.e., the shape measurement data.

The representative value obtaining unit 210c is a functional unit that obtains representative distance (representative value), based on the distance between the point of origin and the multiple points calculated by the distance calculation unit 210b, which make up the shape measurement data. In the example as illustrated in FIG. 18, for example, the representative value obtaining unit 210c obtains, as a representative distance, the distance between the point of origin and the point x₂₃ calculated by the distance calculation unit 210b.

As a representative distance to be obtained, for example, the average value or median value of the multiple distances calculated by the distance calculation unit 210b, or the distance between the point of origin and each of the points indicated by the shape measurement data, which is determined in advance not to involve any noise from among several items of shape measurement data selected by the selection unit 210a, may be used. However, such representative distance is not limited to the distance as described above, and representative distance may be obtained using any desired method as long as that distance is suitable for the determination made by the distance determination unit 210d, as will be described later.

The distance determination unit 210d is a functional unit that determines whether the difference between each a plurality of distances calculated by the distance calculation unit 210b and a representative distance obtained by the representative value obtaining unit 210c satisfies a predetermined condition. A predetermined condition is, for example, a condition as to whether the above difference exceeds a predetermined threshold. In such cases, in FIG. 18, the distance determination unit 210d determines that the difference between the distance between the point of origin and the point x₂₃, which is representative distance, and the distance between the point of origin and each one of the points x₂₄ and x₂₅ exceeds a predetermined threshold.

The removal unit 210e is a functional unit that determines that the shape measurement data that is determined by the distance determination unit 210d to dissatisfy a predetermined condition involves noise and removes that shape measurement data as noise. For example, as illustrated in FIG. 18, the removal unit 210e determines that the shape measurement data that corresponds to the points x₂₄ and x₂₅, which is determined by the distance determination unit 210d not to satisfy a predetermined condition in regard to the distance from the point of origin involves noise, and removes the shape measurement data as noise.

For example, the noise reduction unit 210 may be implemented by a program executed by the CPU 101 as illustrated in FIG. 2.

Note also that the noise reduction unit 210 may reduce or remove noise every time the acquisition unit 203 obtains shape measurement data. Alternatively, the noise reduction unit 210 may reduce or remove noise after a predetermined amount of the obtained shape measurement data is accumulated.

Note also that all of or some of the coordinate system setting unit 201, the space division unit 202, the acquisition unit 203, the noise reduction unit 210, the determination unit 204, the evaluation value calculation unit 205, and the display control unit 207 may be implemented by hardware circuitry such as a field-programmable gate array (FPGA) or an application specific integrated circuit (ASIC), in place of a software program.

The coordinate system setting unit 201, the space division unit 202, the acquisition unit 203, the noise reduction unit 210, the determination unit 204, the evaluation value calculation unit 205, the input unit 206, the display control unit 207, and the display unit 208 merely indicate functions schematically, and no limitation is intended by such configurations. For example, a plurality of functional units that are illustrated as independent functional units in FIG. 17 may be configured as a single functional unit. Alternatively, a function of a single functional unit as illustrated in FIG. 17 may be divided into a plurality of functions implemented by a plurality of functional units.

### Processes Performed by Measurement Support Device

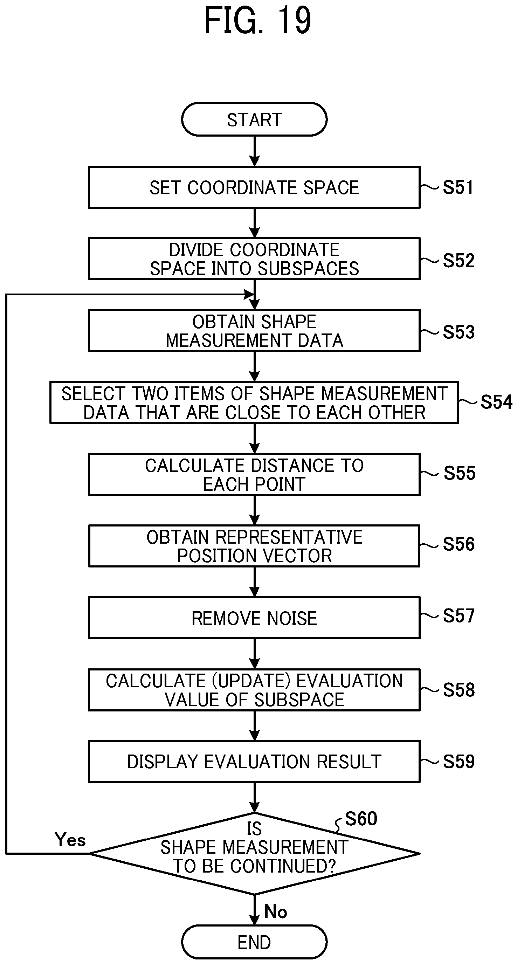

FIG. 19 is a flowchart of the processes performed by the measurement support device 10b according to the second modification of the third embodiment of the present disclosure.

The processes that are performed by the measurement support device 10b according to the present modification of the third embodiment of the present disclosure is described below with reference to FIG. 19.

### Steps S51 to S53

The processes in steps S51 to S53 are equivalent to the processes in the steps S11 to S13 as described above with reference to FIG. 7, respectively. After the processes in the step S53 end, the process shifts to the processes in a step S54.

### Step S54

The selection unit 210a of the noise reduction unit 210 of the measurement support device 10b selects any desired multiple items of shape measurement data that are temporally or spatially close to each other, from among a plurality of items of the shape measurement data obtained by the acquisition unit 203. Then, the process shifts to the processes in a step S55.

### Step S55

The distance calculation unit 210b of the noise reduction unit 210 of the measurement support device 10b calculates the distance (the length of a position vector at each point) between the point of origin (for example, the center point when the object 30 has a spherical shape) and the multiple points calculated by the selection unit 210a, which make up the shape measurement data. Then, the process shifts to the processes in a step S56.

### Step S56

The representative value obtaining unit 210c of the noise reduction unit 210 of the measurement support device 10b obtains representative distance, based on the distance between the point of origin and the multiple points calculated by the distance calculation unit 210b, which make up the shape measurement data. A method of obtaining a representative distance is described as above. Then, the process shifts to the processes in a step S57.

### Step S57

The distance determination unit 210d of the noise reduction unit 210 of the measurement support device 10b determines whether the difference between each a plurality of distances calculated by the distance calculation unit 210b and a representative distance obtained by the representative value obtaining unit 210c satisfies a predetermined condition. For example, the distance determination unit 210d determines whether the above difference exceeds a predetermined threshold. Further, the removal unit 210e of the noise reduction unit 210 of the measurement support device 10b determines that the shape measurement data that is determined by the distance determination unit 210d to dissatisfy a predetermined condition involves noise, and removes the shape measurement data as noise. After all the shape measurement data obtained by the acquisition unit 203 goes through the processes in the steps S54 to S57, the process shifts to the processes in a step S58.

### Steps S58 to S60

The processes in the steps S58 to S60 are equivalent to the processes in the steps S14 to S16 as described above with reference to FIG. 7, respectively.

The processes that are performed by the measurement support device 10b according to the present modification of the third embodiment of the present disclosure are implemented by the processes as in the above steps S51 to S60.

As described above, the measurement support device 10b according to the present modification removes, from the shape measurement data measured by the three-dimensional measuring device 20, the shape measurement data that is determined to involve noise based on the difference between each distance and a representative value. Accordingly, unnecessary groups of points that do not belong to the object to be measured can be removed. Due to such a configuration, noise existing near the object can be removed, and the evaluation value can be calculated and obtained based on valid groups of points (consistent with the purposes of a person who performs measurement) extracted from the shape measurement data measured by the three-dimensional measuring device 20. Accordingly, the progress of shape measurement can appropriately be quantified, and the person who performs the measurement (technician) can easily figure out whether the shape measurement data obtained by measuring the shape of an object is sufficient.

As described above, the processes in the third embodiment of the present disclosure and the first and second modifications of the third embodiment of the present disclosure are executed in an independent manner. However, no limitation is intended thereby, and any of the processes may be combined. For example, after the processes according to the third embodiment of the present disclosure or the processes according to the first modification of the third embodiment are performed to remove the shape measurement data that is determined to involve noise, the processes according to the second modification of the third embodiment may further be performed. Due to such a configuration, noise existing near the object can be removed with greater reliability, and the evaluation value can be calculated and obtained based on further valid groups of points extracted from the shape measurement data.

In the embodiments of the present disclosure and their modifications, when at least some of the multiple functional units of the measurement support device 10 is implemented by executing a program, such a program may be incorporated in advance in a read only memory (ROM) or the like. The program to be executed by the measurement support device 10 according to the embodiments of the present disclosure and their modifications may be installed for distribution in any desired computer-readable recording medium such as a compact disc, a read-only memory (CD-ROM), a flexible disk (FD), a compact disc-recordable (CD-R), and a digital versatile disk (DVD) in a file format installable or executable by a computer. The program executed in the measurement support device 10 according to the embodiments of the present disclosure and their modifications may be provided upon being stored in a computer connected to a network such as the Internet and downloaded through the network. A program to be executed by the measurement support device 10 according to the embodiments of the present disclosure and their modifications may be provided or distributed through a network such as the Internet. A program to be executed by the measurement support device 10 according to the embodiments of the present disclosure and their modifications has module structure including at least one of the above-described functional units. Regarding the actual hardware related to the program, the CPU 101 reads and executes the program from the memory as described above (e.g., the ROM 103 and the auxiliary memory 104) to load the program onto the main memory (e.g., the RAM 102) to implement the above multiple functional units.

Further, as described above, any one of the above-described and other methods of the present disclosure may be embodied in the form of a computer program stored on any kind of storage medium. Examples of storage media include, but are not limited to, flexible disks, hard disks, optical discs, magneto-optical discs, magnetic tape, nonvolatile memory cards, ROM, etc. Alternatively, any one of the above-described and other methods of the present disclosure may be implemented by ASICs, prepared by interconnecting an appropriate network of conventional component circuits, or by a combination thereof with one or more conventional general-purpose microprocessors and/or signal processors programmed accordingly.

The present disclosure can be implemented in any convenient form, for example using dedicated hardware, or a mixture of dedicated hardware and software. The present disclosure may be implemented as computer software implemented by one or more networked processing apparatuses. The network can comprise any conventional terrestrial or wireless communications network, such as the Internet. The processing apparatuses can compromise any suitably programmed apparatuses such as a general purpose computer, personal digital assistant, mobile telephone (such as a WAP or 3G-compliant phone) and so on. Since the present disclosure can be implemented as software, each and every aspect of the present disclosure thus encompasses computer software implementable on a programmable device. The computer software can be provided to the programmable device using any conventional carrier medium. The carrier medium can compromise a transient carrier medium such as an electrical, optical, microwave, acoustic or radio frequency signal carrying the computer code. An example of such a transient medium is a TCP/IP signal carrying computer code over an IP network, such as the Internet. The carrier medium can also comprise a storage medium for storing processor readable code such as a floppy disk, hard disk, CD ROM, magnetic tape device or solid state memory device.

The hardware platform includes any desired kind of hardware resources including, for example, a CPU, a RAM, and a HDD. The CPU may be implemented by any desired kind of any desired number of processor. The RAM may be implemented by any desired kind of volatile or non-volatile memory. The HDD may be implemented by any desired kind of non-volatile memory capable of storing a large amount of data. The hardware resources may additionally include an input device, an output device, or a network device, depending on the type of the apparatus. Alternatively, the HDD may be provided outside of the apparatus as long as the HDD is accessible. In this example, the CPU, such as a cache memory of the CPU, and the RAM may function as a physical memory or a primary memory of the apparatus, while the HDD may function as a secondary memory of the apparatus.

## Claims

1. A measuring apparatus (10) comprising:
an acquisition unit (203) configured to obtain data indicating position coordinates of a surface of an object (30); and
a control unit (207) configured to output information to associate amount information of the data belonging to at least one of a plurality of subspaces (401, 402, 403, 404, 411, 412, 413, 414, 415, 416, 417, 418) obtained by dividing a coordinate space with the plurality of subspaces (401, 402, 403, 404, 411, 412, 413, 414, 415, 416, 417, 418) when the amount information and the plurality of subspaces (401, 402, 403, 404, 411, 412, 413, 414, 415, 416, 417, 418) are displayed.

2. The measuring apparatus (10) according to claim 1, further comprising
a noise reduction unit (209, 210) configured to remove, from the data obtained by the acquisition unit (203), the data determined to involve noise based on a prescribed condition, as noise,
wherein the control unit (207) outputs information to associate amount information of the data from which the noise has been removed by the noise reduction unit (209, 210), belonging to the plurality of subspaces (401, 402, 403, 404, 411, 412, 413, 414, 415, 416, 417, 418), with display of the plurality of subspaces (401, 402, 403, 404, 411, 412, 413, 414, 415, 416, 417, 418).

3. The measuring apparatus (10) according to claim 2,
wherein the noise reduction unit (209, 210)
selects two items of data that are temporally or spatially close to each other, from among a plurality of items of the data obtained by the acquisition unit (203),
sets a displacement vector formed by the two items of data and a position vector determined by a prescribed point and one of the two items of data, and
determines that at least one of the selected two items of data involves noise, based on an angle that the displacement vector forms with the position vector, and removes the noise.

4. The measuring apparatus (10) according to claim 2,
wherein the noise reduction unit (209, 210)
selects three items of data that are temporally or spatially close to each other, from among a plurality of items of the data obtained by the acquisition unit (203),
selects any desired two pairs of data from among the three items of data and sets two displacement vectors determined by the selected two pairs of data, and
determines that at least one of the selected three items of data involves noise, based on an angle that the two displacement vectors form, and removes the noise.

5. The measuring apparatus (10) according to claim 2,
wherein the noise reduction unit (209, 210)
selects a plurality of items of data that are temporally or spatially close to each other, from among a plurality of items of the data obtained by the acquisition unit (203),
calculates a plurality of distances between a predetermined point and each of a plurality of points indicated by the plurality of items of data,
obtains a representative value from the plurality of calculated distances, and
determines, when a difference between one of the plurality of distances and the representative value exceeds a predetermined threshold, that the data corresponding to the one of the plurality of distances involves noise, and removes the noise.

6. The measuring apparatus (10) according to any one of claims 1 to 5, further comprising:
a construction unit (201) configured to construct the coordinate space; and
a division unit (202) configured to divide the coordinate space constructed by the construction unit (201) into the plurality of subspaces (401, 402, 403, 404, 411, 412, 413,414,415,416,417,418),
wherein the acquisition unit (203) obtains, as the data, shape measurement data indicating coordinate data of a surface shape of the object (30) from a measuring device (20),
the measuring apparatus (10) further comprising
a determination unit (204) configured to determine to which one of the plurality of subspaces (401, 402, 403, 404, 411, 412, 413, 414, 415, 416, 417, 418) the shape measurement data obtained by the acquisition unit (203) belongs,
wherein the control unit (207) causes a display unit (208) to display amount information of the shape measurement data that makes up a plurality of subareas (501, 502, 503, 504, 511, 512, 513, 514) belonging to the plurality of subspaces (401, 402, 403, 404, 411, 412, 413, 414, 415, 416, 417, 418) of a measured object (30a, 30b, 31a, 32a, 33a) represented by the shape measurement data.

7. The measuring apparatus (10) according to claim 6, wherein the control unit (207) causes the display unit (208) to display the data so as to be visually identifiable for each one of the plurality of subareas (501, 502, 503, 504, 511, 512, 513, 514).

8. The measuring device (10) according to claim 6 or 7, further comprising
a calculation unit (205) configured to calculate an evaluation value based on the shape measurement data that makes up the plurality of subareas (501, 502, 503, 504, 511, 512, 513, 514), for each one of the plurality of subspaces (401, 402, 403, 404, 411, 412, 413, 414, 415, 416, 417, 418) including the plurality of subareas (501, 502, 503, 504, 511, 512,513,514),
wherein, as a mode of controlling the display unit (208) to display the data, the control unit (207) causes the display unit (208) to display the plurality of subareas (501, 502, 503, 504, 511, 512, 513, 514) of the measured object (30a, 30b, 31a, 32a, 33a) based on the evaluation value.

9. The measuring apparatus (10) according to claim 8, wherein the calculation unit (205) calculates, as the evaluation value, an integrated value of a number of points of the shape measurement data belonging to the plurality of subspaces (401, 402, 403, 404, 411, 412, 413, 414, 415, 416, 417, 418).

10. The measuring apparatus (10) according to claim 8, wherein the calculation unit (205) calculates, as the evaluation value, a ratio of an integrated value of a number of points of the shape measurement data belonging to the plurality of subspaces (401, 402, 403, 404, 411, 412, 413, 414, 415, 416, 417, 418) with reference to a prescribed reference value.

11. The measuring apparatus (10) according to claim 8, wherein the calculation unit (205) calculates and obtains, as the evaluation value, a density of the shape measurement data belonging to the plurality of subspaces (401, 402, 403, 404, 411, 412, 413,414,415,416,417,418).

12. The measuring apparatus (10) according to any one of claims 8 to 11,
wherein, as a mode of controlling the display unit (208) to display the data, the control unit (207) color-codes the plurality of subareas (501, 502, 503, 504, 511, 512, 513, 514) corresponding to the evaluation value with a color associated with the evaluation value, and causes the display unit (208) to display a color-coded plurality of subareas (501, 502, 503, 504, 511, 512, 513, 514).

13. The measuring apparatus (10) according to any one of claims 8 to 11, wherein, as a mode of controlling the display unit (208) to display the data, the control unit (207) causes the display unit (208) to display a graph indicating the evaluation value.

14. The measuring apparatus (10) according to any one of claims 6 to 13,
wherein the construction unit (201) constructs three-dimensional coordinate space as the coordinate space, and
wherein the division unit (202) divides the three-dimensional coordinate space into the plurality of subspaces (401, 402, 403, 404, 411, 412, 413, 414, 415, 416, 417, 418).

15. The measuring apparatus (10) according to claim 14,
wherein the construction unit (201)
extracts a coordinate axis based on a distribution of the shape measurement data obtained by the acquisition unit (203), and
constructs the three-dimensional coordinate space based on the extracted coordinate axis.
